(19) Europäisches Patentamt
European Patent Office
Office européen des brevets

(11) **EP 3 892 181 B1**

(12) **EUROPÄISCHE PATENTSCHRIFT**

(45) Veröffentlichungstag und Bekanntmachung des Hinweises auf die Patenterteilung:
**02.08.2023   Patentblatt 2023/31**

(21) Anmeldenummer: **21150014.5**

(22) Anmeldetag: **13.11.2017**

(51) Internationale Patentklassifikation (IPC):
***A47L 15/00*** (2006.01)      ***A47L 15/24*** (2006.01)
***A61L 2/18*** (2006.01)

(52) Gemeinsame Patentklassifikation (CPC):
**A47L 15/241; A47L 15/0049; A61L 2/18;**
A47L 15/0076; A47L 2401/023; A47L 2401/11;
A47L 2401/12; A47L 2401/14; A47L 2401/20;
A47L 2401/34; A47L 2501/04; A47L 2501/05;
A47L 2501/06; A47L 2501/07; A47L 2501/20;

(Forts.)

(54) **VERFAHREN UND REINIGUNGSVORRICHTUNG ZUM REINIGEN VON REINIGUNGSGUT**

METHOD AND CLEANING DEVICE FOR CLEANING ITEMS

PROCÉDÉ ET DISPOSITIF DE NETTOYAGE DESTINÉS AU NETTOYAGE DES ARTICLES À NETTOYER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.11.2016   DE 102016222308**

(43) Veröffentlichungstag der Anmeldung:
**13.10.2021   Patentblatt 2021/41**

(62) Dokumentnummer(n) der früheren Anmeldung(en) nach Art. 76 EPÜ:
**17801419.7 / 3 537 944**

(73) Patentinhaber: **MEIKO Maschinenbau GmbH & Co. KG**
**77652 Offenburg (DE)**

(72) Erfinder:
• **Gaus, Bruno**
  **77654 Offenburg (DE)**
• **Näger, Thomas**
  **77652 Offenburg (DE)**

• **Peukert, Thomas**
  **77815 Bühl (Baden) (DE)**
• **Jakway, Allen**
  **77736 Zell am Harmersbach (DE)**
• **Kasper, Pamela**
  **77731 Willstätt (DE)**
• **Röderer, Werner**
  **77948 Friesenheim (DE)**

(74) Vertreter: **Altmann Stößel Dick Patentanwälte PartG mbB**
**Theodor-Heuss-Anlage 2**
**68165 Mannheim (DE)**

(56) Entgegenhaltungen:
EP-A1- 2 241 240      EP-B1- 2 053 959
WO-A1-2006/097294      DE-A1-102013 226 637

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)
A47L 2501/24; A47L 2501/30; A61G 9/02;
A61L 2/07; A61L 2202/17

**Beschreibung**

Gebiet der Erfindung

Die Erfindung betrifft ein Verfahren und eine Reinigungsvorrichtung zum Reinigen von Reinigungsgut, insbesondere für den Einsatz in der gewerblichen Geschirrspültechnik und/oder in Großküchen. Reinigungsvorrichtungen der genannten Art können beispielsweise in Einrichtungen zur Gemeinschaftsverpflegung eingesetzt werden, wie insbesondere in Betriebskantinen, Kantinen in Schulen, Behörden, Krankenhäusern oder Pflegeeinrichtungen. Die Reinigungsvorrichtung kann insbesondere zur Reinigung von Reinigungsgut in Form von Spülgut eingesetzt werden, welches direkt oder indirekt zur Zubereitung, Aufbewahrung oder Darreichung von Speisen und Getränken eingesetzt wird. Insbesondere kann es sich dabei um Geschirr und/oder Tabletts handeln. Auch andere Einsatzgebiete der vorliegenden Erfindung sind grundsätzlich denkbar, insbesondere Einsatzgebiete mit grundsätzlich beliebigem Spülgut.

Stand der Technik

Aus dem Stand der Technik sind eine Vielzahl von Reinigungsvorrichtungen, auch als Reinigungsgeräte bezeichnet, bekannt, welche Reinigungsgut reinigen und/oder desinfizieren können. Die Erfindung nimmt im Folgenden, ohne Einschränkung anderer möglicher Einsatzgebiete, maßgeblich Bezug auf die gewerbliche Spültechnik, beispielsweise die gewerbliche Geschirrspültechnik. So sind, neben Programmspülmaschinen mit statischem Spülverfahren, beispielsweise Transportspülmaschinen oder Durchlaufspülmaschinen bekannt, bei welchen das Reinigungsgut mittels einer Transportvorrichtung durch eine oder mehrere Reinigungskammern transportiert wird. Die Ausgestaltung dieser Reinigungsvorrichtungen hängt insgesamt stark von den verschiedenen Randbedingungen, wie beispielsweise der Art des zu reinigenden Reinigungsguts, den Verschmutzungen, dem Durchsatz oder ähnlichen Bedingungen ab. Exemplarisch kann auf Reinigungsvorrichtungen verwiesen werden, welche beispielsweise in DE 10 2004 056 052 A1, in DE 10 2007 025 263 A1 oder in DE 10 2013 220 035 A1 beschrieben sind.

Im Gegensatz zur Haushalts-Geschirrspültechnik weisen gewerbliche Spülmaschinen in der Regel mehrere Fluidtanks auf, um einen Durchsatz an Reinigungsgut beschleunigen zu können. Beispielsweise ist neben dem eigentlichen Waschtank in der Regel noch ein Nachspültank vorgesehen, in welchem Nachspüloder Klarspülfluid bereits temperiert werden kann, während noch der Hauptwaschvorgang läuft. Transportspülmaschinen weisen in der Regel eine Mehrzahl an Reinigungszonen auf, in welchen das Reinigungsgut sequenziell gereinigt wird. Mit dieser Erhöhung des Durchsatzes ist jedoch in der Regel eine deutliche Erhöhung an Energieaufwand und in vielen Fällen auch eine Erhöhung eines Bedarfs an Reinigerlösung verbunden im Vergleich zur Haushalts-Geschirrspültechnik. Eine allgemein erstrebenswerte Verringerung des Energiebedarfs und des Bedarfs an Reinigerlösung oder allgemein an Tensiden oder anderen chemischen Zusätzen darf jedoch nicht zu Lasten eines Reinigungsergebnisses und insbesondere der erzielten Hygienewirkung erfolgen. So sind für die gewerbliche Spültechnik verschiedene Hygiene-Standards vorgesehen wie beispielsweise in Deutschland die DIN 10510 oder in den USA der so genannte NSF3-Standard. Letzterer schreibt beispielsweise vor, dass bestimmte Temperaturen und Konzentrationen von Desinfektionsmitteln in der Klarspülung eingehalten werden müssen und dass bei Abweichungen eine Warnung ausgegeben werden muss.

EP 2 053 959 B1 beschreibt ein Verfahren zur Beurteilung und Sicherstellung der thermischen Hygienewirkung in einer Mehrtankspülmaschine. Mittels mindestens eines Temperatursensors wird die Temperatur innerhalb mindestens einer Behandlungszone an eine Maschinensteuerung übermittelt. Anhand der Temperatur wird ein Wärmeeintrag auf das Spülgut ermittelt und dieser Wärmeeintrag mit einem vorgegebenen Wärmeeintrag verglichen. Die Transportgeschwindigkeit wird variiert, um den tatsächlichen Wärmeeintrag zu regeln.

Aus DE 10 2006 014 464 B3 ist eine Vorrichtung zum Reinigen von Geschirr bekannt, welche eine Transporteinrichtung für das Reinigungsgut aufweist. Diese kann das Reinigungsgut mit unterschiedlichen Transportgeschwindigkeiten durch mindestens eine Reinigungskammer transportieren. Weiterhin ist eine Dosiereinrichtung für Reinigungsmittel vorgesehen, die eine bestimmte Menge an Reinigungsmittel in die Reinigungskammer abgibt. Die Dosiereinrichtung wählt die Menge an abzugebendem Reinigungsmittel abhängig von der Transportgeschwindigkeit.

DE 10 2006 019 546 A1 beschreibt eine Dosiereinrichtung für einen Spülautomaten, mit einer Abgabeeinheit und einem Regler für die abzugebende Menge an Reiniger, der die Abgabeeinheit ansteuert. Die Dosiereinrichtung weist eine Steuereinheit auf, die eine Ansteuerung der Abgabeeinheit durch den Regler für bestimmte Zeitintervalle unterbricht und/oder drosselt oder verstärkt.

EP 1 886 615 B1 beschreibt eine Geschirrspülmaschine, die so konstruiert ist, dass Essgeschirr mit Waschwasser gewaschen wird. Das Waschwasser ist in einem Waschwassertank enthalten und wird nach dem Waschen in dem Waschwassertank gesammelt. Ein Teil des in dem Waschwassertank enthaltenen Waschwassers wird nach jedem Waschzyklus durch zugeführtes Wasser ersetzt. Die Geschirrspülmaschine weist weiterhin eine Spülmittelzuführvorrichtung auf, die dem Waschwasser ein Spülmittel zuführt und die eine Zuführmenge des Spülmittels auf der Grundlage der Anzahl der Waschzyklen erhöht.

DE 10 2013 203 933 A1 offenbart ein Verfahren zur Reinigung von Reinigungsgut, bei welchem das Reinigungsgut mit mindestens einer Spülflüssigkeit beaufschlagt wird. Eine Zufuhr mindestens einer Komponente der Spülflüssigkeit wird dabei von einem Anfangswert mit zunehmender Reinigungsdauer auf einen Gleichgewichtswert erhöht.

DE 10 2014 102 970 A1 beschreibt eine Transportspülmaschine zum Spülen von Spülgut. In dieser ist mindestens eine Klarspülzone mit mindestens einer Klarspüldüse zum Sprühen von Klarspülflüssigkeit auf das Spülgut und eine der Klarspülzone zugeordnete Dosiereinrichtung zum Zudosieren eines Desinfektionsmittels zu der Klarspülflüssigkeit vorgesehen. Weiterhin ist eine Sensoreinrichtung vorgesehen, welche ausgelegt ist zum Detektieren der Desinfektionsmittelkonzentration in der in der Klarspülzone versprühten Klarspülflüssigkeit. Ferner ist eine Steuereinrichtung vorgesehen, welche ausgelegt ist, den mit der Sensoreinrichtung detektierten Wert der Desinfektionsmittelkonzentration mit vorab festlegbaren Konzentrationswerten zu vergleichen und, bei zu hohen Abweichungen der Desinfektionsmittelkonzentration von den vorab festlegbaren Konzentrationswerten, den Spülvorgang zu unterbrechen oder ein Warnsignal an einen Bediener auszugeben.

In DE 10 2009 024 569 A1 wird ein Verfahren zum Betrieb einer Transportspülmaschine beschrieben. Die Transportspülmaschine weist mindestens eine Vorspülzone mit mindestens einer Vorspüldüse zum Sprühen von Vorspülflüssigkeit auf das Spülgut und mindestens eine Klarspülzone mit mindestens einer Klarspüldüse zum Sprühen von Klarspülflüssigkeit auf das Spülgut auf. Zumindest ein Teil der versprühten Klarspülflüssigkeit wird als Vorspülflüssigkeit wiederverwendet. Eine der Klarspülzone zugeordnete Dosiereinrichtung ist vorgesehen zum Zudosieren eines Desinfektionsmittels zu der Klarspülflüssigkeit. Weiterhin ist zusätzlich eine der Vorspülzone zugeordnete Dosiereinrichtung vorgesehen, zum Zudosieren eines Desinfektionsmittels zu der Vorspülflüssigkeit. Ferner ist eine Sensoreinrichtung vorgesehen, welche ausgelegt ist zum Detektieren der Desinfektionsmittelkonzentration in der in der Vorspülzone versprühten Vorspülflüssigkeit. Ferner ist eine Steuereinrichtung vorgesehen, welche ausgelegt ist, in Abhängigkeit von der mit der Sensoreinrichtung detektierten Desinfektionsmittelkonzentration eine der Dosiereinrichtungen derart anzusteuern, dass Desinfektionsmittel zudosiert wird.

DE 10 2011 007 507 A1 beschreibt eine Transportspülmaschine mit mindestens einer Waschzone und mindestens einer Klarspülzone sowie mit einer Transportvorrichtung zum Transportieren von Spülgut durch die mindestens eine Waschzone und die mindestens eine Klarspülzone. Um den Verbrauch an Desinfektionschemikalie und Energie zu reduzieren, aber dennoch ein optimales Klarspülergebnis zu erhalten, ist erfindungsgemäß vorgesehen, dass die in der Klarspülzone versprühte Klarspülflüssigkeit eine zudosierte Desinfektionschemikalie aufweist, und dass mindestens eine der Klarspülzone nachgeschaltete zusätzliche Klarspülzone vorgesehen ist zum Sprühen von Frischwasser ohne Desinfektionschemikalie auf das zu behandelnde Spülgut. Der mindestens einen zusätzlichen Klarspülzone ist eine Auffangeinrichtung zugeordnet zum Auffangen von in der zusätzlichen Klarspülzone versprühter Flüssigkeit. Ferner ist ein Flüssigkeitstransfersystem vorgesehen, über welches die in der Auffangeinrichtung aufgefangene Flüssigkeit direkt der Waschzone zuführbar ist.

DE 10 2012 213 271 A1 beschreibt eine Spülmaschine, welche als Transportspülmaschine oder als Programmautomat ausgebildet ist und mindestens ein Waschsystem zum Versprühen von Waschflüssigkeit in einer Waschzone bzw. während einer Waschphase und mindestens ein Klarspülsystem zum Versprühen von Klarspülflüssigkeit in einer Klarspülzone bzw. während einer Klarspülphase aufweist, wobei ferner eine Steuereinrichtung vorgesehen ist zum Ansteuern des mindestens einen Waschsystems und/oder des mindestens einen Klarspülsystems gemäß einem vorab festgelegten Ablaufprogramm. Um zu erreichen, dass Ressourcen beim Betrieb der Spülmaschine eingespart werden können, wobei gleichzeitig die in den jeweils lokal (territorial begrenzt) geltenden Hygienerichtlinien festgelegten Anforderungen an den hygienischen Betrieb der Spülmaschine erfüllt werden, ist erfindungsgemäß vorgesehen, dass in der Steuereinrichtung mindestens ein erstes Ablaufprogramm abgelegt ist, welches unter Berücksichtigung von in einem ersten territorialen Bereich geltenden Anforderungen an einen hygienischen Betrieb einer gewerblichen Spülmaschine erstellt wurde, und dass in der Steuereinrichtung mindestens ein zweites Ablaufprogramm abgelegt ist, welches unter Berücksichtigung von in einem zweiten territorialen Bereich geltenden Anforderungen an einen hygienischen Betrieb einer gewerblichen Spülmaschine erstellt wurde.

DE 10 2008 024 543 A1 beschreibt ein Verfahren zum Betreiben einer Spülmaschine. Dabei wird während eines Waschprozesses eine Waschpumpe derart angesteuert, dass zumindest zeitweise über das Leitungssystem eine Waschflüssigkeit mindestens einer Waschdüse zugeführt wird. Weiterhin wird ein Verlauf eines hydrostatischen Druckes der Waschflüssigkeit in einem Leitungssystem erfasst und mit einem vorgegebenen Druckverlauf verglichen. Bei einer Abweichung des erfassten Druckverlaufes von dem vorgegebenen Druckverlauf wird in Abhängigkeit von der Größe und dem zeitlichen Gradienten einer Differenz zwischen dem vorgegebenen Druckverlauf und dem erfassten Druckverlauf automatisch regelnd auf den Waschprozess eingewirkt, über eine optische und/oder akustische Schnittstelle der Spülmaschine eine Fehlermeldung ausgegeben oder über eine Remote-Control-Schnittstelle der Spülmaschine eine Meldung an eine Fernwartungsstelle ausgegeben.

WO 2011/062790 A2 offenbart eine Spülvorrichtung, bei welcher ein Sensor und eine Steuerungsvorrichtung zur Detektion einer volumetrischen Flussrate einer Flüssigkeit in einem Rohrsystem und zum Vergleich eines Profils derselben mit einem Profil einer vorgegebenen Flussrate vorgesehen sind. Die Steuerungsvorrichtung ist eingerichtet, um im Falle einer Abweichung automatisch regelnd einzugreifen oder eine Warnung auszugeben.

Weiter bekannt sind WO 2006/097294A1, EP 2 241 240 A1, DE 10 2013 226 637 A1, US 6,615,850 B1, DE 10 2004 049 392 A1, DE 10 2007 021 245 A1, US 4,439,242, US 5,603,233, US 5,820,691, US 3,896,827, EP 2 228 000 A2, US 2016/0309981 A1, EP 1 824 373 B1, EP 0 621 810 B1, US 2016/0206767 A1, DE 69519474 T2.

Diese bekannten Verfahren und Reinigungsvorrichtungen mit thermisch-chemischen Verfahren beschreiben Messung und Variation der Temperatur, um eine gewünschte Hygienewirkung zu erzielen und Hygiene-Standards einzuhalten. Derartige Verfahren und Reinigungsvorrichtung erlauben keine genaue Steuerung und Überwachung der Hygienewirkung. Eine ausreichende Hygienesicherheit kann zudem nur unter großem Ressourceneinsatz von Energie und chemischen Zusätzen erreicht werden, welches zu hohen Aufwendungen für den Betreiber führt und nachteilig für die Umwelt ist.

Aufgabe der Erfindung

Es ist daher Aufgabe der vorliegenden Erfindung, ein Verfahren und eine Reinigungsvorrichtung bereitzustellen, welche die Nachteile bekannter Verfahren und Reinigungsvorrichtungen zumindest weitgehend vermeiden. Insbesondere soll eine Verbesserung der Hygienesicherheit bei niedrigem Ressourceneinsatz ermöglicht werden.

Offenbarung der Erfindung

Diese Aufgabe wird gelöst durch ein Verfahren und eine Reinigungsvorrichtung zum Reinigen von Reinigungsgut mit den Merkmalen der unabhängigen Patentansprüche. Vorteilhafte Weiterbildungen, welche einzeln oder in beliebiger Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nichtausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne Einschränkung der Möglichkeit, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten, die hierdurch eingeleiteten Merkmale mit anderen Merkmalen zu kombinieren, seien es optionale oder nicht-optionale Merkmale, unangetastet bleiben.

In einem ersten Aspekt der vorliegenden Erfindung wird ein Verfahren zum Reinigen von Reinigungsgut vorgeschlagen. Unter einem Reinigungsgut können dabei allgemein beliebige Güter verstanden werden, welche einer Reinigung oder einem Reinigungsverfahren unterzogen werden können. Ohne Beschränkung weiterer möglicher Ausgestaltungen wird dabei im Folgenden Bezug genommen auf Reinigungsgut in Form von Spülgut. Spülgut soll dabei jegliche Gegenstände umfassen, welche zur Zubereitung, Darreichung oder Aufbewahrung von Speisen und Getränken vorgesehen sind. Als Beispiele sind hierbei Geschirr wie Tassen, Teller, Gläser, Schalen oder Schüsseln zu nennen. Weiterhin sind Töpfe, Tabletts, Besteck, Warmhaltevorrichtungen oder ähnliche Vorrichtungen zu nennen. Es wird jedoch ausdrücklich darauf hingewiesen, dass auch andere Arten von Reinigungsgut gereinigt werden können, wie beispielsweise industrielles Stückgut, Schüttgut, Container oder andere Arten von Reinigungsgut.

Unter Reinigen kann sowohl eine Befreiung des Reinigungsguts von anhaftendem Schmutz oder anderen Verunreinigungen als auch eine keimreduzierende und/oder keimabtötende Wirkung oder sogar desinfizierende Wirkung verstanden werden. Unter einer Hygienisierung wird grundsätzlich sowohl eine Befreiung von Keimen, insbesondere eine keimreduzierende und/oder keimabtötende oder sogar desinfizierende Wirkung, als auch eine Befreiung von Substanzen wie Chemikalien, beispielsweise Rückstände von Zusätzen des mindestens einen Reinigungsfluids, verstanden.

In dem Verfahren wird eine Reinigungsvorrichtung mit mindestens einer Reinigungskammer und mindestens einer Beaufschlagungsvorrichtung zum Beaufschlagen des Reinigungsguts in der Reinigungskammer mit mindestens einem Reinigungsfluid verwendet. Unter einer Reinigungsvorrichtung ist allgemein im Rahmen der vorliegenden Erfindung eine Vorrichtung zu verstehen, welche eingerichtet ist, um Reinigungsgut zumindest teilweise von anhaftenden Verunreinigungen und/oder Keimen zu befreien. Die Reinigungsvorrichtung kann beispielsweise eine Geschirrspülmaschine sein, insbesondere eine gewerbliche Geschirrspülmaschine, beispielsweise eine Programm-Geschirrspülmaschine und/oder eine Durchlaufgeschirrspülmaschine. Alternativ oder zusätzlich kann die Reinigungsvorrichtung jedoch auch ganz oder teilweise als Reinigungs- und Desinfektionsgerät ausgestaltet sein, beispielsweise als Reinigungsvorrichtung, welche zur Reinigung von Behältern zur Aufnahme menschlicher Ausscheidungen eingerichtet ist. Allgemein kann diesbezüglich beispielsweise auf die in DE 10 2004 056 052 A1 und/oder in DE 10 2007 025 263 A1 beschriebenen Reinigungsvorrichtungen verwiesen werden. Die Reinigungsvorrichtung kann auch eine Spülmaschine sein, wie sie zur Reinigung von Behältern im Umfeld der Lebensmittel-Produktion und/oder Lebensmittel-Verarbeitung zum Einsatz kommen kann. Weiter kann es sich bei der Reinigungsvorrichtung um eine Desinfektionsspülmaschine handeln, beispielsweise eine Spülmaschine zur Reinigung und Desinfektion von Atemmasken. Auch andere Ausgestaltungen sind jedoch grundsätzlich denkbar.

Die Reinigungsvorrichtung kann insbesondere als Transportspülmaschine, insbesondere als Durchlaufgeschirrspülmaschine, ausgestaltet sein. Unter einer Transportgeschirrspülmaschine ist dabei eine Geschirrspülmaschine zu verstehen, also eine Maschine zur Reinigung von Spülgut in Form von Geschirr, welche eingerichtet ist, um das Spülgut durch eine Reinigungskammer zu transportieren. Insbesondere kann es sich dabei um eine Bandtransport-Geschirrspülmaschine und/oder eine Korbtransport-Geschirrspülmaschine handeln, also eine Geschirrspülmaschine, bei welcher das Geschirr mittels eines Transportbandes durch die Reinigungsvorrichtung transportiert wird, beispielsweise eines Transportbandes, auf welches das Geschirr unmittelbar aufgesetzt wird und/oder auf welches einer oder mehrere Körbe mit dem zu reinigenden Geschirr aufgesetzt werden. Die Transportgeschirrspülmaschine kann insbesondere für den gewerblichen Einsatz ausgestaltet sein, beispielsweise in einer oder mehreren der oben genannten Einrichtungen zur Gemeinschaftsverpflegung. Auch andere Arten von Reinigungsvorrichtungen sind jedoch grundsätzlich möglich

Das Reinigungsfluid kann beispielsweise eine Reinigungsflüssigkeit und/oder ein gasförmiges Reinigungsfluid sein. Beispielsweise kann dieses Reinigungsfluid eine Reinigungsflüssigkeit umfassen, beispielsweise eine wässrige Reinigungsflüssigkeit, beispielsweise Wasser in Form von Frischwasser und/oder mit einem oder mehreren Zusatzstoffen, beispielsweise mit einem oder mehreren Reinigerkonzentraten und/oder einem oder mehreren Klarspülerkonzentraten und/oder einem oder mehreren Desinfektionsmitteln. Beispielsweise kann das Reinigungsfluid einen Zusatz oder mehrere Zusatzstoffe aufweisen, beispielsweise mindestens einen Zusatzstoff ausgewählt aus der Gruppe bestehend aus einem Reinigerkonzentrat, einem Klarspüler und einem Desinfektionsmittel. Alternativ oder zusätzlich kann das Reinigungsfluid beispielsweise Dampf umfassen. Auch andere Ausgestaltungen sind j e-doch grundsätzlich denkbar. Das mindestens eine Reinigungsfluid kann grundsätzlich beispielsweise mindestens eine Reinigungsflüssigkeit umfassen, insbesondere mindestens eine wässrige Reinigungsflüssigkeit. Auch andere Arten von Reinigungsfluiden sind grundsätzlich einsetzbar. Unter einem Reinigungsfluid kann somit also ein beliebiges Fluid, insbesondere eine Flüssigkeit, verstanden werden, welche eine reinigende Wirkung auf das Reinigungsgut ausüben kann.

Unter einer Reinigungskammer ist dabei allgemein eine Kammer zu verstehen, in welcher der oben beschriebene Reinigungsvorgang des Reinigungsguts vollständig oder teilweise durchgeführt wird. Insbesondere kann in einer Reinigungskammer die Beaufschlagung mit dem Reinigungsfluid oder einem von mehreren Reinigungsfluids erfolgen. Weiterhin kann die Reinigungsvorrichtung mindestens eine Transportvorrichtung umfassen, welche eingerichtet ist, um das Reinigungsgut in einer Transportrichtung von einem Einlaufbereich durch die Reinigungskammer zu einem Auslaufbereich zu transportieren. Die Kammer ist vorzugsweise durch ein Gehäuse vollständig oder teilweise umschlossen. Insbesondere kann die Reinigungskammer tunnelartig ausgestaltet sein oder einen Teil eines Tunnels umfassen, beispielsweise mit einem Einlauf und einem Auslauf, wobei das Reinigungsgut am Einlauf in die Reinigungskammer eingeführt wird und am Auslauf aus der Reinigungskammer austritt. Unter einem Einlauf ist also ein Bereich außerhalb der Reinigungskammer zu verstehen, welcher in Transportrichtung unmittelbar vor der Reinigungskammer angeordnet ist und in welchem das Reinigungsgut auf die Transportvorrichtung aufgebracht werden kann. Entsprechend ist unter einem Auslauf ein Bereich außerhalb der Reinigungskammer zu verstehen, welcher in Transportrichtung unmittelbar hinter der Reinigungskammer angeordnet ist und in welchem das Reinigungsgut von der Transportvorrichtung entnommen werden kann. Unter einer Transportvorrichtung ist allgemein im Rahmen der vorliegenden Erfindung eine beliebige Vorrichtung zu verstehen, welche eingerichtet ist, um das Reinigungsgut in der Transportrichtung durch die Reinigungskammer zu transportieren. Beispielsweise kann diese Transportvorrichtung ausgewählt sein aus einer Bandtransportvorrichtung mit mindestens einem Transportband, einem Klinkentransportsystem und einer Rollentransportvorrichtung mit mindestens einer Transportrolle oder einer Mehrzahl von Transportrollen, beispielsweise einer oder mehreren angetriebenen Transportrollen, mittels derer beispielsweise das Reinigungsgut direkt und/oder ein oder mehrere das Reinigungsgut aufnehmende Körbe in der Transportrichtung durch die Reinigungskammer transportiert werden können. Die Transportvorrichtung kann beispielsweise mindestens einen Antrieb aufweisen, beispielsweise mindestens einen Antriebsmotor. Beispielsweise kann es sich hierbei um einen Antrieb handeln, welcher mindestens ein Transportband und/oder mindestens eine Transportrolle oder eine andere Art von Transportelement der Transportvorrichtung antreibt.

Unter einer Beaufschlagungsvorrichtung ist dabei grundsätzlich eine beliebige Vorrichtung oder Kombination von Vorrichtungen zu verstehen, mittels derer die Beaufschlagung des Reinigungsguts mit dem Reinigungsfluid erfolgen kann, beispielsweise durch ein Besprühen, Bestrahlen oder Betropfen des Reinigungsguts mit dem Reinigungsfluid. Beispielsweise kann die Beaufschlagungsvorrichtung mindestens ein Düsensystem aufweisen. So können beispielsweise in der Transportgeschirrspülmaschine ein oder mehrere Düsensysteme vorgesehen sein. Sind beispielsweise mehrere Reinigungszonen vorgesehen, so kann jeder Reinigungszone beispielsweise mindestens ein derartiges Düsensystem zugeordnet sein. Beispielsweise kann die Reinigungsvorrichtung derart eingerichtet sein, dass das Reinigungsgut die Reinigungszonen nacheinander passiert. Beispielsweise kann für eine Anordnung derartiger Reinigungszonen auf den oben genannten Stand der Technik verwiesen werden.

Das Verfahren umfasst die folgenden Schritte:

a. Vorgabe eines bei der Reinigung zu erreichenden Soll-Hygienewerts;
b. zeitaufgelöste Erfassung mindestens zweier Einflussgrößen, welche einen Einfluss auf eine Hygienisierung des Reinigungsguts haben;
c. Bestimmen von Hygienewertanteilen aus den Einflussgrößen unter Verwendung eines vorgegebenen Zusammenhangs zwischen jeder Einflussgröße und deren jeweiligem Hygienewertanteil;
d. Ermittlung eines voraussichtlichen Ist-Hygienewerts am Ende der Reinigung aus den Hygienewertanteilen;

e. Vergleich des voraussichtlichen Ist-Hygienewerts mit dem Soll-Hygienewert; und
f. Beeinflussung mindestens einer Einflussgröße in Abhängigkeit von dem Ergebnis des Vergleichs.

Die Verfahrensschritte können in der genannten Reihenfolge oder auch in einer anderen Reihenfolge durchgeführt werden. Weiterhin können auch einer oder mehrere der genannten Verfahrensschritte zeitlich parallel, zeitlich überlappend oder auch gleichzeitig durchgeführt werden. Weiterhin können einer oder mehrere der Verfahrensschritte einzeln oder gruppenweise wiederholt oder dauerhaft durchgeführt werden.

Unter einem Hygienewert ist allgemein eine Größe zu verstehen, welche ein Hygiene-Ergebnis quantifiziert. Beispielsweise kann der Hygienewert ein Hygienelevel sein. Beispielsweise kann der Hygienewert ausgewählt sein aus A0-Werten oder H.U.E.-Werten; einem Maß für eine Reduktion einer Population eines Zielkeims. A0-Werte und H.U.E.-Werte sind ein Maß für die Reduktion, bzw. Absterberate, eines Zielkeims durch eine thermische Einwirkung. Je höher die einwirkende Temperatur, desto größer ist die keimreduzierende Wirkung pro Zeiteinheit. Beispielsweise lassen sich bei höherer Temperatur mehr A0-Werte und H.U.E.-Werte pro Sekunde erreichen. Wünschenswert kann jedoch ein Betrieb der Reinigungsvorrichtung bei niedrigen Temperaturen sein, beispielsweise zur Reduzierung eines Einsatzes von Ressourcen wie Energie zum Heizen. Die Reduktion des Zielkeims kann durch weitere Größen beeinflusst werden, wie beispielsweise die Einflussgrößen, Mechanik und Chemie, insbesondere durch eine Konzentration oder Zusammensetzung mindestens eines Zusatzstoffes des Reinigungsfluids. Ein SUE-Wert (sanitation unit equivalent) pro Sekunde kann ein Maß für die Reduktion des Zielkeims sein, welches eine Abhängigkeit von der Temperatur und der verwendeten Reinigerchemie berücksichtigt, wobei

$$SUE/sec = Ae^{BT},$$

wobei A = 1/7000000 und B = 0,191. Ein SUE-Wert von 100 entspricht dabei einer 5 log-Stufen Reduktion des Zielkeims. Die Parameter A und B sind experimentelle Werte, bestimmt unter Verwendung einer Population von Staphylococcus aureus als Zielkeimpopulation. Das Verfahren ist nicht an die genannte Zielkeimpopulation gebunden. Beispielsweise kann das Verfahren an geänderte Anforderungen angepasst werden und auch andere Keime als Zielkeim sind vorstellbar, beispielsweise Enterococcus faecium. Das SUE-Verfahren kann eine Berücksichtigung der Konzentration oder Zusammensetzung des mindestens einen Zusatzstoffes des Reinigungsfluids auf die Hygienewirkung erlauben und so bei gleichbleibender Hygienewirkung einen Betrieb der Reinigungsvorrichtung bei niedrigen Temperaturen ermöglichen. Bevorzugt kann der Hygienewert ein SUE-Wert sein.

Weiter kann bei dem Hygienewert zwischen einem Soll- und einem Ist-Hygienewert unterschieden werden. Unter einem Soll-Hygienewert ist ein Hygiene-Ergebnis zu verstehen, welches die Reinigungsvorrichtung bei der Reinigung erreichen soll, insbesondere an einem Ende des Reinigungsvorgangs, beispielsweise an einem zeitlichen Ende eines Reinigungsprogramms in einer Einkammerspülmaschine und/oder einer Programmspülmaschine und/oder beispielsweise an einem Ende eines Tunnels bei einer Transportgeschirrspülmaschine. Unter einem Ist-Hygienewert ist ein Hygiene-Ergebnis zu verstehen, welches die Reinigungsvorrichtung tatsächlich erreicht.

Der Soll-Hygienewert kann ausgewählt sein aus der Gruppe bestehend aus: einem bei der Reinigung insgesamt zu erreichenden AO-Wert nach DIN EN ISO 15883; einem zu erreichenden Hygienelevel nach DIN 10510, bei den Sollvorgaben von Tank- und Boiler Temperaturen nach DIN 10510; einer bei der Reinigung insgesamt zu erreichenden Anzahl an HUE-Einheiten nach dem NSF3-Standard; einer bei der Reinigung insgesamt zu erreichenden Reduktion einer Population eines Zielkeims, insbesondere einer bei der Reinigung insgesamt zu erreichenden Reduktion der Population von Staphylococcus aureus. Der Soll-Hygienewert kann einem Absterben einer Population von Staphylococcus aureus um 99,999% entsprechen. Insbesondere kann der Soll-Hygienewert ein SUE-Wert sein. Unter einer Vorgabe eines bei der Reinigung zu erreichenden Soll-Hygienewerts kann ein Bereitstellen eines Soll-Hygienewerts verstanden werden. Beispielsweise kann die Vorgabe eine Auswahl eines Soll-Hygienewerts, beispielsweise aus einer Liste, und/oder eine Eingabe eines Soll-Hygienewerts, beispielsweise in eine Mensch-Maschine-Schnittstelle der Reinigungsvorrichtung, insbesondere einer Steuerung der Reinigungsvorrichtung, umfassen.

Unter einer Einflussgröße wird dabei allgemein im Rahmen der vorliegenden Erfindung eine quantifizierbare Größe verstanden, welche in irgendeiner Weise den Betrieb der Reinigungsvorrichtung und/oder ein Reinigungsergebnis der Reinigungsvorrichtung beeinflussen kann. Die Hygienisierung, auch als Hygienewirkung bezeichnet, für Reinigungsabläufe kann durch verschiedene Einflussgrößen bestimmt werden. Die Einflussgrößen können ausgewählt sein aus der Gruppe bestehend aus: einer Dauer der Reinigung; mindestens einer Temperatur des mindestens einen Reinigungsfluids; mindestens einer eine mechanische Einwirkung des mindestens einen Reinigungsfluids auf das Reinigungsgut charakterisierenden Messgröße, insbesondere einem Druck und/oder einer Flussrate des mindestens einen Reinigungsfluids; mindestens einer Konzentration mindestens eines Desinfektionsmittels in dem mindestens einen Reinigungsfluid; mindestens einer Konzentration mindestens eines Reinigerkonzentrats in dem mindestens einen Reinigungsfluid; mindestens einem pH-Wert des mindestens einen Reinigungsfluids; mindestens einer eine Wirkung einer Klarspülung charakterisierenden Messgröße. Die Einflussgrößen können sich gegenseitig beeinflussen. Die Hygienewirkung kann als ein Kreisdiagramm dargestellt werden, in welchem die Einflussgrößen Kreissegmente darstellen, welche sich gegenseitig zum gesamten Kreis ergänzen müssen. Eine Verminderung einer dieser Einflussgrößen kann jeweils durch eine Erhöhung einer oder mehrerer der anderen Einflussgrößen kompensiert werden.

Die mindestens zwei Einflussgrößen können als erste Einflussgröße mindestens eine Temperatur des mindestens einen Reinigungsfluids sowie weiterhin mindestens eine weitere Einflussgröße umfassen. Unter einer Erfassung einer Einflussgröße wird allgemein eine Bestimmung und/oder Detektion und/oder Messung der Einflussgröße, insbesondere eines Messwerts, und/oder eines Maßes für die Einflussgröße verstanden. Unter einer zeitaufgelösten Erfassung der Einflussgröße wird allgemein eine zeitliche Erfassung und/oder zeitliche Entwicklung der Einflussgröße verstanden. Die zeitaufgelöste Erfassung der mindestens zwei Einflussgrößen kann zu vorgegebenen oder vorgebbaren Zeiten oder in vorgegebenen oder vorgebbaren Abständen erfolgen. Insbesondere kann die zeitaufgelöste Erfassung zeitlich vor einer Beeinflussung der Einflussgröße erfolgen.

Beispielsweise kann die Reinigungsvorrichtung mindestens einen Sensor zur Erfassung der Einflussgrößen aufweisen. Unter einem Sensor ist dabei im Rahmen der vorliegenden Erfindung allgemein eine Vorrichtung zu verstehen, welche eingerichtet ist, um einen oder mehrere Messwerte der Einflussgrößen zu erfassen, vorzugsweise in Form eines oder mehrerer elektronischer Messwerte. Die Messwerte können analog und/oder digital ausgestaltet sein. Der Sensor kann beispielsweise mindestens ein Sensorelement umfassen, welches eingerichtet ist, um entsprechend der zu erfassenden Zustandsvariablen mindestens ein Sensorsignal zu erzeugen, beispielsweise ein analoges oder digitales Sensorsignal. Zusätzlich zu dem mindestens einen Sensorelement kann der Sensor jedoch ein oder mehrere weitere Elemente umfassen. So kann der Sensor insbesondere eine oder mehrere Zuleitungen umfassen, welche mit dem Sensorelement und/oder anderen Elementen des Sensors verbunden sind. Weiterhin kann beispielsweise mindestens eine Ansteuer- und/oder Auswerteschaltung vorgesehen sein, welche mit dem Sensorelement verbunden sein kann und welche häufig auch als Messplatine oder einfach nur als Platine bezeichnet wird. Darüber hinaus kann der Sensor weitere Elemente umfassen, wie beispielsweise mindestens einen Analog-Digital-Wandler (A/D-Wandler) und/oder mindestens eine Signalleitung und/oder Datenleitung und/oder eine Schnittstelle. Die Gesamtheit, die sich aus dem mindestens einen Sensorelement und optional dem mindestens einen weiteren Element des Sensors zusammensetzt, kann zusammen ein Sensorsystem und/oder eine Messkette des Sensors bilden, welche letztendlich mit einer Steuerung verbunden sein kann. Der Sensor selbst kann also ein Sensorsystem sein oder kann Bestandteil eines Sensorsystems sein. Die Messkette und deren Ausgestaltung können letztendlich bestimmen, wie sich ein bestimmter Wert der Einflussgrößen und/oder eine Änderung dieses Wertes auf ein oder mehrere Sensorsignale auswirken, welche der Steuerung zur Verfügung gestellt werden. Der Sensor kann also insgesamt mindestens ein Sensorelement umfassen, sowie mindestens ein weiteres Element, insbesondere mindestens ein weiteres Element ausgewählt aus der Gruppe umfassend: eine Ansteuer- und/oder Auswerteschaltung (insbesondere zur Aufbereitung mindestens eines Messwertes oder Sensorwertes), eine Schnittstelle, einen Analog-Digital-Wandler (A/D-Wandler), ein drahtloses und/oder drahtgebundenes Übertragungselement, eine Signalleitung und/oder Datenleitung, eine Spannungsversorgung, einen A/D-Wandler, ein Anzeigeelement, einen Datenspeicher, ein Funkmodul. Auch andere Ausgestaltungen sind jedoch möglich.

Basis für die Durchführung des vorliegenden Verfahrens kann eine entsprechende Steuerungshardware und/oder Steuerungssoftware der Reinigungsvorrichtung sein, welche vollständig oder teilweise in der Steuerung implementiert sein kann oder welche auch zumindest teilweise in anderen Bestandteilen der Reinigungsvorrichtung integriert sein kann.

Beispielsweise kann der Sensor mindestens einen Temperatursensor umfassen, insbesondere mindestens ein Sensorelement in Form beispielsweise eines temperaturabhängigen Widerstands, beispielsweise eines NTC und/oder eines PTC.

Die Reinigungsvorrichtung kann eingerichtet sein, einen aktuellen Wert einer Dosierung eines Desinfektionsmittels in dem mindestens einen Reinigungsfluid und/oder mindestens eine Konzentration des mindestens eines Reinigerkonzentrats in dem mindestens einen Reinigungsfluid und/oder mindestens einen pH-Wert zu bestimmen. Beispielsweise kann die Reinigungsvorrichtung eingerichtet sein, eine Reinigerkonzentration in einer Spülflüssigkeit zu bestimmen. Beispielsweise kann die Reinigungsvorrichtung eingerichtet sein, eine Zusammensetzung des Reinigers, insbesondere einen pH-Wert der Spülflüssigkeit im Umwälztank, zu bestimmen. Die Reinigungsvorrichtung kann mindestens einen Sensor ausgewählt aus der Gruppe bestehend aus einer Leitwertelektrode, einer pH-Sonde, einer Redox-Sonde, einen Chlorsensor aufweisen. Eine Erfassung kann beispielsweise in einem Waschtank der Reinigungsvorrichtung erfolgen. Insbesondere kann eine Erfassung in einem Waschtank der Reinigungsvorrichtung erfolgen, in welchem eine Verfälschung der Messwerte durch einen Eintrag von Schmutz-Bestandteilen, beispielsweise Kochsalz, gering ist, beispielsweise in einem letzten Waschtank. Eine Erfassung einer Konzentration eines Reinigungskonzentrats kann über eine einer Reinigungszone zugeführte Reinigermenge und eine Regenerationswassermenge erfolgen. Die Reinigungsvorrichtung kann mindestens einen Durchflusssensor aufweisen, und/oder die Reinigermenge kann mittels einer Waage bestimmt werden. Die Regenerationswassermenge kann beispielsweise aus einer Laufzeit einer Flüssigkeitstransfereinrichtung bestimmt werden.

Die Reinigungsvorrichtung kann mindestens einen Sensor zur Erfassung mindestens einer eine mechanische Einwirkung des mindestens einen Reinigungsfluids auf das Reinigungsgut charakterisierenden Größe, insbesondere eines Drucks und/oder einer Flussrate des mindestens einen Reinigungsfluids, aufweisen. Beispielsweise kann der Sensor mindestens ein Sensorelement in Form eines Drucksensors umfassen, beispielsweise in Form eines mikromechanischen Drucksensors. Der Drucksensor kann eingerichtet sein, einen Systemdruck in einem Spülsystem der Reinigungsvorrichtung zu erfassen. Beispielsweise kann der Sensor einen Prallplattensensor aufweisen, welcher eine Wirkung einzelner Düsen, so genannter Referenzdüsen, erfassen kann. Auch andere Ausgestaltungen sind jedoch denkbar.

Unter einem Hygienewertanteil ist allgemein ein Hygienewert pro Zeitabschnitt zu verstehen. Der Hygienewertanteil kann ein zu erreichender Wert (Soll- Wert) sein. Der Hygienewertanteil kann ein tatsächlich erreichter Wert (Ist- Wert) sein. Unter einem Zeitabschnitt kann insbesondere eine Zeitdauer und/oder eine Zeiteinheit verstanden werden. Ein erreichter Ist-Hygienewert in einem Zeitraum kann durch Aufsummieren der Hygienewertanteile in dem Zeitraum bestimmt werden. Beispielsweise können die Hygienewertanteile über die Länge einer Behandlungszone bzw. die Zeitdauer, für die sich das Reinigungsgut innerhalb dieser Zone befindet, aufsummiert werden und so ein Hygienewertanteil für die Behandlungszone bestimmt werden. Der Hygienewertanteil für die Behandlungszone kann als Hygienewertanteil, den diese Zone zum gesamten Ergebnis beiträgt, bezeichnet werden. Alternativ oder zusätzlich kann ein Hygienewertanteil für einen festen Zeitabschnitt betrachtet werden, beispielsweise ein Hygienewertanteil pro Sekunde. Der Hygienewertanteil kann eine Hygienisierungsrate umfassen. Unter einer Hygienisierungsrate ist eine zeitliche Änderung zu verstehen. Der Zusammenhang zwischen den Einflussgrößen und deren jeweiligen Hygienewertanteilen kann vorgegeben sein durch mindestens eine der folgenden Vorgaben: mindestens einer Kalibrationskurve, insbesondere mindestens einer Kurve zu einer Hygienewirkung, wobei die Kalibrationskurve jeweils einen Zusammenhang zwischen dem jeweiligen Hygienewertanteil der Einflussgröße und einer weiteren Einflussgröße aufzeigt, wobei die mindestens eine weitere Einflussgröße konstant gehalten wird; mindestens einer Kurvenschar mit einer Mehrzahl von Kalibrationskurven, wobei die Kalibrationskurven jeweils einen Zusammenhang zwischen dem jeweiligen Hygienewertanteil der Einflussgröße und einer weiteren Einflussgröße aufzeigen, wobei innerhalb jeder Kalibrationskurve der Kurvenschar die mindestens eine weitere Einflussgröße konstant gehalten wird, wobei sich die mindestens eine weitere Einflussgröße zwischen den Kalibrationskurven der Kurvenschar unterscheidet; mindestens einer Kalibrationstabelle, wobei die Kalibrationstabelle jeweils einen Zusammenhang zwischen dem jeweiligen Hygienewertanteil der Einflussgröße und einer weiteren Einflussgröße aufzeigt, wobei die mindestens eine weitere Einflussgröße konstant gehalten wird; mindestens einer Tabellenschar an Kalibrationstabellen mit einer Mehrzahl von Kalibrationstabellen, wobei die Kalibrationstabellen jeweils einen Zusammenhang zwischen dem jeweiligen Hygienewertanteil der Einflussgröße und einer weiteren Einflussgröße aufzeigen, wobei innerhalb jeder Kalibrationstabelle der Tabellenschar die mindestens eine weitere Einflussgröße konstant gehalten wird, wobei sich die mindestens eine weitere Einflussgröße zwischen den Kalibrationstabellen der Tabellenschar unterscheidet. Auf diese Weise kann eine Abhängigkeit der einzelnen Einflussgrößen zueinander bestimmt werden. Bei einer Berücksichtigung mindestens zweier Einflussgrößen, insbesondere aller Einflussgrößen, kann ein mehrdimensionales Feld bestimmt werden, in welchem, wie weiter unten detailliert beschrieben, die Reinigungsvorrichtung, insbesondere die Steuerung der Reinigungsvorrichtung die einzelnen Einflussgrößen variieren kann.

Das Verfahren kann mindestens eine Kalibration umfassen. Bei der Kalibration kann der Zusammenhang zwischen jeder Einflussgröße und deren jeweiligem Hygienewertanteil bestimmt werden. Beispielsweise kann die Kalibration einmalig, beispielsweise bei einer ersten Inbetriebnahme der Reinigungsvorrichtung erfolgen. Beispielsweise kann die Kalibration wiederholt erfolgen, beispielsweise in vorgebbaren oder wählbaren Zeitintervallen. Der Zusammenhang kann nach der Kalibration in mindestens einem Datenspeicher, beispielsweise der Steuerung, gespeichert werden. Beispielsweise kann der Zusammenhang in einer Tabelle gespeichert werden, wobei beispielsweise die Kalibrationskurven als diskrete Werte gespeichert werden. Bei der Kalibration kann bestimmt werden, wie lange unter jeweiligen konstanten Bedingungen, einschließlich vorgegebener konstanter Werte der Einflussgrößen, benötigt wird, bis der vorgegebene Soll-Hygienewert erreicht wird, insbesondere ein vorgegebener Reduktionsfaktor oder eine vorgegebene Reduktion mindestens eines Zielkeims, wobei aus dieser Information über die Zeitdauer der Hygienewertanteil bestimmt wird, unter Berücksichtigung eines anfänglichen Hygienewerts, des Soll-Hygienewerts und der Zeitdauer.

Unter einem voraussichtlichen Ist-Hygienewert wird allgemein ein erwartetes Hygieneergebnis verstanden, welches am Ende der Reinigung erreicht wird. Die Ermittlung des voraussichtlichen Ist-Hygienewertes kann eine Berücksichtigung und/oder ein Aufsummieren aller bisher, also bis zum Zeitpunkt der Ermittlung des voraussichtlichen Ist-Hygienewerts, erreichten Hygienewertanteile umfassen. Insbesondere können alle seit einem Startzeitpunkt eines Reinigungsprogramms auf das Reinigungsgut wirkenden Hygienewertanteile berücksichtigt und/oder aufsummiert werden. Der voraussichtliche Ist-Hygienewert kann eine Prognose für den Ist-Hygienewert am Ende der Reinigung sein. Der voraussichtliche Ist-Hygienewert am Ende der Reinigung kann aus den Hygienewertanteilen bestimmt werden unter der Annahme, dass die aktuellen Einflussgrößen für den Rest der Reinigung unverändert bleiben. Der voraussichtliche Ist-Hygienewert am Ende der Reinigung kann aus den Hygienewertanteilen berechnet werden unter Verwendung mindestens eines Algorithmus, ausgewählt aus der Gruppe bestehend aus: einer Integration der Summe der Hygienewertanteile der Einflussgrößen über die voraussichtliche restliche Dauer der Reinigung; einer Gesamtheit der zeitlichen Riemann-Summen der jeweiligen Hygienewertanteile der Einflussgrößen über die voraussichtliche restliche Dauer der Reinigung. Unter einem Ende einer Reinigung kann ein zeitliches Ende in einer Einkammermaschine und/oder Programmspülmaschine und/oder ein Tunnelende bei einer Transportspülmaschine verstanden werden.

Das Verfahren umfasst einen Vergleich des voraussichtlichen Ist-Hygienewerts mit dem Soll-Hygienewert. Unter einem Vergleich des voraussichtlichen Ist-Hygienewerts mit dem Soll-Hygienewert wird allgemein ein Bestimmen einer Gleichheit oder einer Abweichung, insbesondere eines Überschreitens und/oder Unterschreitens, des voraussichtlichen Ist-Hygienewerts und des Soll-Hygienewert verstanden. Der Vergleich kann einen mathematischen Vergleich umfassen. Insbesondere kann Verfahrensschritt e. eine Erkennung umfassen, ob der voraussichtliche Ist-Hygienewert kleiner ist als der Soll-Hygienewert.

Das Verfahren umfasst eine Beeinflussung mindestens einer Einflussgröße in Abhängigkeit von dem Ergebnis des Vergleichs. Insbesondere kann die Reinigungsvorrichtung eingerichtet sein, mindestens eine Einflussgröße derart zu beeinflussen, dass ein tatsächlich am Ende der Reinigung erreichter Ist-Hygienewert dem Soll-Hygienewert entspricht.

Unter einer Beeinflussung wird allgemein eine Änderung und/oder Variation und/oder Anpassung und/oder Regelung mindestens einer Einflussgröße verstanden. Die Beeinflussung der mindestens einen Einflussgröße kann zu vorgegebenen oder vorgebbaren Zeiten erfolgen. Beispielsweise kann ein Zeitpunkt zur Beeinflussung in einem Datenspeicher der Steuerung der Reinigungsvorrichtung hinterlegt sein. Die Steuerung kann eingerichtet sein, einen Zeitpunkt der Beeinflussung vorzugeben. Beispielsweise kann ein Zeitpunkt der Beeinflussung ausgewählt werden, insbesondere durch die Steuerung und/oder einen Benutzer. Die Beeinflussung kann zu einem Zeitpunkt während eines Reinigungsprogramms erfolgen. Beispielsweise kann der Vergleich des voraussichtlichen Ist-Hygienewerts und des Soll-Hygienewerts an einem Ende eines Reinigungsschrittes des Reinigungsprogramms erfolgen und die Beeinflussung der mindestens einen Einflussgröße in einem anschließenden Reinigungsschritt erfolgen. Beispielsweise kann in einer Transportspülmaschine die Beeinflussung an einem vorgegebenen Zeitpunkt während des Transports des Reinigungsguts, beispielsweise an einem bestimmten Ort, insbesondere zwischen zwei Reinigungszonen, erfolgen. Wie oben ausgeführt, kann, beispielsweise in einem Kalibrationsschritt, ein Zusammenhang zwischen dem jeweiligen Hygienewertanteil der Einflussgröße und einer weiteren Einflussgröße bestimmt werden, beispielsweise in Form einer Kurvenschar. Die Reinigungsvorrichtung, insbesondere die Steuerung, kann eingerichtet sein, eine Abhängigkeit der einzelnen Einflussgrößen, insbesondere mindestens zweier Einflussgrößen, zueinander zu bestimmen. Bevorzugt ist die Reinigungsvorrichtung, insbesondere die Steuerung, eingerichtet, eine Abhängigkeit jeder einzelnen Einflussgröße zu jeder anderen Einflussgröße zu bestimmen. Die Reinigungsvorrichtung kann eingerichtet sein, ein mehrdimensionales Feld zu bestimmen, in welchem die einzelnen Einflussgrößen variieren werden können. Für jede Einflussgröße kann eine Beeinflussung nur innerhalb vorgegebener und/oder vorgebbarer Grenzen erfolgen.

Beispielsweise kann die Dauer der Reinigung verändert werden, wenn bei dem Vergleich erkannt wird, dass der voraussichtliche Ist-Hygienewert von dem Soll-Hygienewert abweicht. Insbesondere kann die Dauer der Reinigung erhöht werden, wenn erkannt wird, dass der voraussichtliche Ist-Hygienewert kleiner ist als der Soll-Hygienewert. Insbesondere kann die Dauer der Reinigung reduziert werden, wenn erkannt wird, dass der voraussichtliche Ist-Hygienewert größer ist als der Soll-Hygienewert. Es kann mindestens eine Maßnahme zur Veränderung der Dauer der Reinigung eingesetzt werden, ausgewählt aus der Gruppe bestehend aus: mindestens eine Programmdauer mindestens eines Reinigungsprogramms wird verändert; mindestens eine Transportgeschwindigkeit mindestens einer Transportvorrichtung, mittels derer das Reinigungsgut durch die mindestens eine Reinigungskammer transportiert wird, wird verändert. Beispielsweise kann eine Programmlaufzeit erhöht oder verkürzt werden. Beispielsweise kann eine Einwirkdauer erhöht oder verkürzt werden. Bei einer Transportspülmaschine kann beispielsweise eine Änderung einer Transportgeschwindigkeit einer Transsportvorrichtung, beispielsweise eines Förderbandes erfolgen. Beispielsweise kann die Transportgeschwindigkeit erhöht oder verlangsamt werden. Die Transportgeschwindigkeit kann bis hin zu einem diskontinuierlichen Betrieb, bei welchem die Transportvorrichtung angehalten wird, reduziert werden. Bei einem diskontinuierlichen Betrieb können Bereiche der Transportvorrichtung, insbesondere des Transportbandes, in einer Reinigungszone, beispielsweise der Frischwasserspülung, verbleiben, so dass die Einwirkzeit verlängert wird. Beispielsweise kann bei Reinigungsvorrichtungen mit Thermodesinfektion bei Unterschreitung einer bestimmten Temperatur die Transportvorrichtung angehalten werden. Beispielsweise kann bei Reinigungsvorrichtungen mit chemischer Desinfektion bei Fehlen der Desinfektionskomponente die Transportvorrichtung angehalten werden. Die Transportgeschwindigkeit kann zudem reduziert werden, wenn die zeitaufgelöste Erfassung der mindestens einen Einflussgröße nicht erfolgt ist und/oder ein Sensorsignal nicht von dem mindestens einen Sensor zur Erfassung der Einflussgröße erzeugt und/oder an die Steuerung übermittelt wurde und/oder die Steuerung, insbesondere der Datenspeicher, keine Information und/oder keinen Wert der Einflussgröße aufweist.

Beispielsweise kann mindestens eine Konzentration mindestens eines Zusatzstoffs in dem mindestens einen Reinigungsfluid verändert werden, wenn erkannt wird, dass der voraussichtliche Ist-Hygienewert von dem Soll-Hygienewert abweicht. Die mindestens eine Konzentration des mindestens einen Zusatzstoffs in dem mindestens einen Reinigungsfluid kann erhöht werden, wenn erkannt wird, dass der voraussichtliche Ist-Hygienewert den Soll-Hygienewert unterschreitet. So kann beispielsweise eine geringere Hygienewirkung aus dem thermischen Einfluss, beispielsweise bei Absenkung einer Temperatur des Reinigungsfluids, beispielsweise einer Spülflüssigkeit in einem Umwälztank, durch eine Erhöhung der Konzentration des mindestens eines Zusatzstoffs in dem mindestens einen Reinigungsfluid und/oder, wie weiter unten beschrieben, eine Veränderung der Zusammensetzung mindestens eines Zusatzstoffs in dem mindestens einen Reinigungsfluids kompensiert werden, ohne eine Laufzeit, insbesondere eine Spülzeit, zu verlängern. Der Zusatzstoff kann mindestens einen Stoff umfassen, ausgewählt aus der Gruppe bestehend aus einem Reinigerkonzentrat und einem Desinfektionsmittel, insbesondere Chlor. Andere Desinfektionsmittel können beispielsweise Brom, Jod oder Wasserstoffperoxid sein oder enthalten. Der Ausdruck Reiniger wird im Rahmen der vorliegenden Anmeldung gleichbedeutend mit dem Ausdruck Zusatzstoff verwendet.

Beispielsweise kann eine Erhöhung eines Anteils und/oder eine zusätzliche Dosierung von Desinfektionskomponenten erfolgen. Beispielsweise kann die Steuerung eingerichtet sein, mindestens eine Dosiervorrichtung der Reinigungsvorrichtung häufiger und/oder für eine längere Zeitdauer zu aktivieren. Beispielsweise kann die Steuerung eingerichtet sein, bei einem kontinuierlichen Lauf der Dosiervorrichtung ein Fördervolumen der Dosiervorrichtung zu erhöhen. In einer Ausführungsform kann die Reinigungsvorrichtung eine erste und eine zweite Dosiervorrichtung aufweisen. Die erste Dosiervorrichtung kann kontinuierlich, beispielsweise mit einer Grundeinstellung betrieben werden. Die Steuerung kann eingerichtet sein, im Fall einer Beeinflussung der Konzentration und/oder Dosierung die zweite Dosiervorrichtung zu aktivieren und so zusätzliches Zusatzmittel zuzuführen. Dieses ist insbesondere vorteilhaft, da die erste und zweite Dosiervorrichtung einfach ausgestaltet sein können. Die durch die erste und zweite Dosiervorrichtung zugeführten Zusatzmittel können sich unterscheiden. Beispielsweise kann ein Zusatzmittel der zweiten Dosiervorrichtung eine Desinfektionskomponente enthalten. So kann insbesondere eine Reinigung bei niedrigen Temperaturen ermöglicht werden. Beispielsweise kann ein Benutzer eine niedrige Temperatur wählen und/oder die Reinigungsvorrichtung kann eingerichtet sein, zu erkennen, dass bei niedrigen Temperaturen gespült werden kann, und Temperaturen in dem Reinigungsprozess abzusenken.

Das Verfahren kann einen Detektionsschritt umfassen, in welchem, insbesondere vor einer Veränderung einer Konzentration und/oder Dosierung, eine Bestimmung einer aktuellen Konzentration und/oder Dosierung erfolgt. Beispielsweise kann in dem Detektionsschritt mindestens ein, oben beschriebener, Sensor verwendet werden, welcher eingerichtet ist, den aktuellen Wert einer Dosierung eines Desinfektionsmittels in dem mindestens einen Reinigungsfluid und/oder mindestens eine Konzentration des mindestens einen Reinigerkonzentrats in dem mindestens einen Reinigungsfluid und/oder mindestens einen pH-Wert zu bestimmen. Die Änderung der Konzentration und/oder Dosierung kann innerhalb einer vorgebbaren und/oder vorgegebenen Grenze erfolgen. Beispielsweise kann eine Obergrenze für eine Dosierung 4 g Reiniger pro Liter Tankinhalt sein.

Beispielsweise kann mindestens eine mechanische Beaufschlagung des Reinigungsguts mit dem mindestens einen Reinigungsfluid verändert werden, wenn bei dem Vergleich erkannt wird, dass der voraussichtliche Ist-Hygienewert von dem Soll-Hygienewert abweicht. Insbesondere kann eine Intensität der mechanischen Beaufschlagung erhöht werden, wenn erkannt wird, dass der voraussichtliche Ist-Hygienewert kleiner ist als der Soll-Hygienewert. Insbesondere kann eine Intensität der mechanischen Beaufschlagung erniedrigt werden, wenn erkannt wird, dass der voraussichtliche Ist-Hygienewert größer ist als der Soll-Hygienewert. Beispielsweise kann mindestens eine Maßnahme zur Veränderung der Intensität der mechanischen Beaufschlagung eingesetzt werden, ausgewählt aus der Gruppe bestehend aus: mindestens ein Druck des mindestens einen Reinigungsfluids wird verändert; mindestens eine Flussrate des mindestens einen Reinigungsfluids wird verändert. Beispielsweise kann eine Erhöhung der mechanischen Wirkung durch ein Zuschalten von weiteren Beaufschlagungsvorrichtungen, beispielsweise weiterer Düsen, erfolgen. Beispielsweise kann eine Pumpleistung der Reinigungsvorrichtung erhöht werden, beispielsweise durch eine Verwendung von einer Reihenschaltung von Pumpen oder mindestens eines Frequenzumrichters.

Das Verfahren kann einen Detektionsschritt umfassen, in welchem, insbesondere vor einer Veränderung einer mechanischen Wirkung, eine Bestimmung einer aktuellen mechanischen Wirkung erfolgt. Wie oben ausgeführt, kann die Reinigungsvorrichtung Sensoren aufweisen, welche eingerichtet sind, die mechanische Wirkung des Spülsystems, insbesondere der Beaufschlagungsvorrichtung, zu überwachen. Die Sensoren zur Überwachung der mechanischen Wirkung können eingerichtet sein, Fehlfunktionen des Spülsystems zu erkennen. Die Sensoren zur Überwachung der mechanischen Wirkung können eingerichtet sein, zu erkennen, dass ein Systemdruck nicht ausreicht und/oder dass einzelne Düsen einen zu geringen oder keinen Wasserstrahl produzieren. Die Sensoren zur Überwachung der mechanischen Wirkung können eingerichtet sein, derartige Detektionsergebnisse der Steuerung zu übermitteln. Die Steuerung kann eingerichtet sein, eine andere Einflussgröße als die mechanische Wirkung zu beeinflussen, um Fehlfunktionen des Spülsystems, insbesondere der Beaufschlagungsvorrichtung, auszugleichen, um einen Soll-Hygienewert zu erreichen. Eine Änderung der mechanischen Wirkung kann innerhalb einer vorgebbaren und/oder vorgegebenen Grenze erfolgen. Beispielsweise kann eine Obergrenze für eine mechanische Wirkung dadurch gegeben sein, dass Schäden am Reinigungsgut vermieden werden sollen.

Beispielsweise kann ein Volumenstrom in der Klarspülung erhöht werden, beispielsweise einer Frischwasserklarspülung und/oder einer Pumpenklarspülung. Das Verfahren kann einen Detektionsschritt umfassen, in welchem, insbesondere vor einer Veränderung des Volumenstroms der Klarspülung, eine Bestimmung eines aktuellen Volumenstroms erfolgt. Beispielsweise kann die Reinigungsvorrichtung mindestens einen Sensor aufweisen, welcher eingerichtet ist, einen Durchfluss in der Frischwasserklarspülung zu bestimmen. Beispielsweise kann die Reinigungsvorrichtung mindestens einen Sensor aufweisen, welcher eingerichtet ist, einen Druck und/oder Durchfluss in der Pumpenklarspülung zu erfassen.

Beispielsweise kann eine Erhöhung der Temperatur, insbesondere in einzelnen Tanks und/oder Reinigungszonen, erfolgen. Das Verfahren kann einen Detektionsschritt umfassen, in welchem, insbesondere vor einer Veränderung der Temperatur, eine Bestimmung einer aktuellen Temperatur erfolgt, beispielsweise unter Verwendung eines oder mehrerer Temperatursensoren.

In dem Verfahrensschritt f. kann zur Beeinflussung der mindestens einen Einflussgröße eine Maßnahme zur Beeinflussung eingesetzt werden, wobei die Maßnahme zur Beeinflussung in Abhängigkeit von einer vorgegebenen und/oder vorgebbaren Maßnahmenhierarchie erfolgt. Die Maßnahmenhierarchie kann in der Steuerung hinterlegt sein, beispielsweise in Form eines Programmcodes. Die Maßnahmenhierarchie kann ausgewählt sein unter Berücksichtigung einer Entscheidungsgrundlage ausgewählt aus der Gruppe bestehend aus: Geschwindigkeit, mit der ein Prozess beeinflussbar ist, beispielsweise Trägheit von Sensoren und des Prozesses; realer Aufwand für die Beeinflussung der mindestens einen Einflussgröße, beispielsweise apparativer Aufwand, Kosten und Störanfälligkeit; Kosten für den Benutzer und/oder Betreiber, beispielsweise Kosten für Reinigungschemie. Beispielsweise kann bei Unterschreitung des Soll-Hygienewerts die Temperatur des mindestens einen Reinigungsfluids erhöht werden. Die Reinigungsvorrichtung kann eingerichtet sein zu erkennen, ob nach angeregter Temperaturerhöhung die geforderte Temperatur innerhalb eines vorgegebenen Zeitfensters erreicht wird, und bei Nichterreichen ein Regelung der Dosierung einzuleiten. Eine Beeinflussung der Dosierung kann einen großen Einfluss auf Betriebskosten haben. Zudem kann eine Nachführung der Konzentration länger brauchen als die Beeinflussung anderer Einflussgrößen, so dass eine Veränderung der Dosierung in der Maßnahmenhierarchie hinter der Beeinflussung anderer Einflussgrößen, insbesondere an einem Ende der Maßnahmenhierarchie, angeordnet sein kann. Insbesondere kann so ein so genannter Overkill durch zu viel Reiniger, insbesondere Zusätze, vermieden werden und ein optimaler Einsatz der Reinigungschemie zum Vorteil der Umwelt und Aufwendungen für den Betreiber erreicht werden. Beispielsweise kann eine Beeinflussung in der folgenden Reihenfolge erfolgen:

- Beeinflussung der Dauer der Reinigung;
- Beeinflussung der mechanischen Wirkung;
- Beeinflussung der Wirkung einer Klarspülung;
- Beeinflussung der Konzentration und/oder Dosierung und/oder mindestens einem pH-Wert;
- Beeinflussung der Temperatur des mindestens einen Reinigungsfluids.

In dem Verfahren kann das Reinigungsgut mittels der mindestens einen Transportvorrichtung durch die mindestens eine Reinigungskammer transportiert werden.

Das Reinigungsgut kann eine Mehrzahl an zu reinigenden Gütern umfassen, wobei jedem Gut ein Hygienisierungskonto zugewiesen wird, auf welchem bisherige Hygienisierungseinheiten, denen das Gut unterworfen wurde, kumuliert gespeichert werden. Unter einer Hygienisierungseinheit wird eine Größe verstanden, welche den Einfluss auf die Keimreduktion quantifiziert. Jedem Hygienisierungsprozess kann abhängig von dem Einfluss auf Keimreduktion eine Anzahl von Hygienisierungseinheiten zugeordnet werden. Beispielsweise kann ein Soll-Hygieneergebnis für ein Reinigungsgut 1000 Hygienisierungseinheiten betragen, so dass zu einem Erreichen des Soll-Hygienewerts 1000 Hygienisierungseinheiten benötigt werden. Null Hygienisierungseinheiten kann bedeuten, dass das Gut bisher keinem Hygienisierungsprozess unterworfen wurde. Unter einem Hygienisierungskonto kann grundsätzlich eine gespeicherte Anzahl von kumulierten Hygienisierungseinheiten verstanden werden. Das Hygienisierungskonto kann beispielweise in einem Datenspeicher der Steuerung gespeichert sein. Das Reinigungsgut kann eine Mehrzahl an zu reinigenden Gütern umfassen, wobei die Transportvorrichtung in Abschnitte unterteilt ist, wobei jedem Abschnitt ein Hygienisierungskonto zugewiesen wird, auf welchem bisherige Hygienisierungseinheiten, denen in dem jeweiligen Abschnitt aufgenommenes Gut unterworfen wurde, kumuliert gespeichert werden. Das Hygienisierungskonto kann wieder auf Null gesetzt werden, wenn ein Fehler in dem Reinigungsvorgang festgestellt wird. Beispielsweise kann das Hygienisierungskonto auf Null gesetzt werden, wenn die Reinigungskammer geöffnet wird.

Die Reinigungskammer kann in eine Mehrzahl von Reinigungszonen unterteilt sein, wobei jede Reinigungszone mindestens eine Beaufschlagungsvorrichtung aufweist, wobei das Reinigungsgut mittels der Transportvorrichtung nacheinander durch die Reinigungszonen transportiert wird. Das Verfahren kann mindestens einen Überprüfungsschritt umfassen, wobei der Überprüfungsschritt durchgeführt wird, solange sich das Reinigungsgut innerhalb der letzten Reinigungszone in einer Transportrichtung der Transportvorrichtung befindet. Bei dem Überprüfungsschritt kann der voraussichtliche Ist-Hygienewert nochmals mit dem Soll-Hygienewert verglichen werden und bei einem Unterschreiten des Soll-Hygienewerts mindestens eine Sicherungsmaßnahme ergriffen werden. Die Sicherungsmaßnahme kann mindestens eine Maßnahme umfassen, ausgewählt aus der Gruppe bestehend aus: die Transportvorrichtung wird angehalten; die Transportvorrichtung wird verlangsamt; ein Druck des Reinigungsfluids in der Beaufschlagungsvorrichtung der letzten Reinigungszone wird erhöht; eine Flussrate des Reinigungsfluids in der Beaufschlagungsvorrichtung der letzten Reinigungszone wird erhöht. Die letzte Reinigungszone kann eine Klarspülzone sein, insbesondere eine Frischwasser-Klarspülzone.

In einem weiteren Aspekt wird eine Reinigungsvorrichtung zum Reinigen von Reinigungsgut vorgeschlagen. Die Reinigungsvorrichtung umfasst mindestens eine Reinigungskammer und mindestens eine Beaufschlagungsvorrichtung zum Beaufschlagen des Reinigungsguts in der Reinigungskammer mit mindestens einem Reinigungsfluid. Die Reinigungsvorrichtung umfasst weiterhin:

    I. mindestens einen Datenspeicher zur Speicherung einer Vorgabe eines bei der Reinigung zu erreichenden Soll-Hygienewerts;

    II. mindestens zwei Messvorrichtungen zur zeitaufgelösten Erfassung mindestens zweier Einflussgrößen, welche einen Einfluss auf eine Hygienisierung des Reinigungsguts haben;

    III. mindestens eine Bestimmungsvorrichtung zur Bestimmung von Hygienewertanteilen aus den Einflussgrößen unter Verwendung eines vorgegebenen Zusammenhangs zwischen jeder Einflussgröße und deren jeweiligem Hygienewertanteil;

    IV. mindestens eine Auswertevorrichtung zur Ermittlung eines voraussichtlichen Ist-Hygienewerts an einem Ende der Reinigung aus den Hygienewertanteilen;

    V. mindestens eine Vergleichsvorrichtung zum Vergleich des voraussichtlichen Ist-Hygienewerts mit dem Soll-Hygienewert; und

VI. mindestens eine Beeinflussungsvorrichtung zur Beeinflussung mindestens einer Einflussgröße in Abhängigkeit von dem Ergebnis des Vergleichs.

Die Reinigungsvorrichtung kann insbesondere eingerichtet sein, um ein Verfahren gemäß einer oder mehreren der oben dargestellten Ausgestaltungen durchzuführen. Dementsprechend kann für mögliche Ausgestaltungen der Reinigungsvorrichtung auf die obige Beschreibung verwiesen werden.

Die Reinigungsvorrichtung kann eine Steuerung aufweisen. Der Datenspeicher, die Bestimmungsvorrichtung, die Auswertevorrichtung, die Vergleichsvorrichtung und die Beeinflussungsvorrichtung können Bestandteile der Steuerung ein. Die Steuerung kann mindestens eine Datenverarbeitungsvorrichtung, insbesondere mindestens einen Prozessor, umfassen. Die Datenverarbeitungsvorrichtung kann programmtechnisch eingerichtet sein, um den Einflussgrößen aufgrund des vorgegebenen Zusammenhangs jeweils einen Hygienewertanteil zuzuweisen und um den voraussichtlichen Ist-Hygienewert am Ende der Reinigung aus den Hygienewertanteilen zu bestimmen. Die Datenverarbeitungsvorrichtung kann weiterhin programmtechnisch eingerichtet sein, um den voraussichtlichen Ist-Hygienewert mit dem Soll-Hygienewert zu vergleichen. Die Datenverarbeitungsvorrichtung kann weiterhin programmtechnisch eingerichtet sein, um zu erkennen, ob der voraussichtliche Ist-Hygienewert kleiner ist als der Soll-Hygienewert. Die Datenverarbeitungsvorrichtung kann weiterhin programmtechnisch eingerichtet sein, um die mindestens eine Einflussgröße in Abhängigkeit von dem Ergebnis des Vergleichs zu beeinflussen.

Der Datenspeicher kann mindestens einen Speicher umfassen, ausgewählt aus einem flüchtigen Datenspeicher und einem nicht flüchtigen Datenspeicher.

Unter einer Messvorrichtung wird allgemein eine Vorrichtung verstanden, welche eingerichtet ist, mindestens eine Einflussgröße zeitaufgelöst zu erfassen. Die Messvorrichtungen können mindestens zwei Sensoren umfassen, ausgewählt aus der Gruppe bestehend aus: mindestens einem Temperatursensor zur Erfassung mindestens einer Temperatur des mindestens einen Reinigungsfluids; mindestens einem mechanischen oder fluidischen Sensor zur Erfassung mindestens einer Messgröße einer mechanischen Einwirkung des mindestens einen Reinigungsfluids auf das Reinigungsgut, insbesondere mindestens einen Drucksensor zur Erfassung mindestens eines Drucks des mindestens einen Reinigungsfluids und/oder mindestens einen Flusssensor zur Erfassung mindestens eines Flusses des mindestens einen Reinigungsfluids; mindestens einem Konzentrationssensor zur Erfassung mindestens einer Konzentration mindestens eines Desinfektionsmittels in dem mindestens einen Reinigungsfluid; mindestens einem Konzentrationssensor zur Erfassung mindestens einer Konzentration mindestens eines Reinigerkonzentrats in dem mindestens einen Reinigungsfluid; mindestens einem pH-Sensor zur Erfassung mindestens eines pH-Werts des mindestens einen Reinigungsfluids. Insbesondere kann die Messvorrichtung eingerichtet sein, Verfahrensschritt b. durchzuführen.

Unter einer Bestimmungsvorrichtung wird allgemein eine Vorrichtung verstanden, welche eingerichtet ist, Hygienewertanteile aus den Einflussgrößen unter Verwendung eines vorgegebenen Zusammenhangs zwischen jeder Einflussgröße und deren jeweiligem Hygienewertanteil zu bestimmen. Insbesondere kann die Bestimmungsvorrichtung eingerichtet sein, Verfahrensschritt c. durchzuführen. Unter einer Auswertevorrichtung wird grundsätzlich eine Vorrichtung verstanden, welche eingerichtet ist, einen voraussichtlichen Ist-Hygienewert an einem Ende der Reinigung aus den Hygienewertanteilen zu ermitteln. Unter einer Vergleichsvorrichtung wird grundsätzlich eine Vorrichtung verstanden, welche eingerichtet ist, den voraussichtlichen Ist-Hygienewert mit dem Soll-Hygienewert zu vergleichen. Unter einer Beeinflussungsvorrichtung wird allgemein eine Vorrichtung verstanden, welche eingerichtet ist, mindestens eine Einflussgröße in Abhängigkeit von dem Ergebnis des Vergleichs zu beeinflussen. Wie oben ausgeführt, kann die Reinigungsvorrichtung eine Steuerung aufweisen, wobei die Bestimmungsvorrichtung, die Auswertevorrichtung, die Vergleichsvorrichtung und die Beeinflussungsvorrichtung Bestandteile der Steuerung sind. Beispielsweise umfassen die Bestimmungsvorrichtung, die Auswertevorrichtung, die Vergleichsvorrichtung und die Beeinflussungsvorrichtung Prozessoren, insbesondere Mikroprozessoren.

Die Reinigungsvorrichtung kann ausgewählt sein aus der Gruppe bestehend aus einer Transportspülmaschine und einer Programmspülmaschine.

Das vorgeschlagene Verfahren und die vorgeschlagene Reinigungsvorrichtung haben zahlreiche Vorteile gegenüber bekannten Verfahren und Reinigungsvorrichtungen. So ist eine genaue Steuerung und Überwachung der Hygienewirkung möglich und eine Verbesserung einer Betriebssicherheit unter der Betrachtung der hygienischen Aspekte ist möglich. Insbesondere kann ein so genannter Overkill durch zu viel Reiniger, insbesondere Zusätze, vermieden werden und so ein optimaler Einsatz der Reinigungschemie zum Vorteil der Umwelt und Aufwendungen für den Betreiber erreicht werden. Bei einer Absenkung einer Temperatur der Spülflüssigkeit im Umwälztank kann durch eine Erhöhung der Konzentration des Reinigers und/oder eine Veränderung der Zusammensetzung des Reinigers im Umwälztank eine geringere Hygienewirkung aus dem thermischen Einfluss kompensiert werden, ohne eine Spülzeit zu verlängern.

Zusammenfassend sind im Rahmen der vorliegenden Erfindung folgende Ausführungsformen besonders bevorzugt:

Ausführungsform 1: Verfahren zum Reinigen von Reinigungsgut, wobei eine Reinigungsvorrichtung mit mindestens einer Reinigungskammer und mindestens einer Beaufschlagungsvorrichtung zum Beaufschlagen des Reinigungsguts in der Reinigungskammer mit mindestens einem Reinigungsfluid verwendet wird, wobei das Verfahren folgende Schritte umfasst:

a. Vorgabe eines bei der Reinigung zu erreichenden Soll-Hygienewerts;

b. zeitaufgelöste Erfassung mindestens zweier Einflussgrößen, welche einen Einfluss auf eine Hygienisierung des Reinigungsguts haben;

c. Bestimmen von Hygienewertanteilen aus den Einflussgrößen unter Verwendung eines vorgegebenen Zusammenhangs zwischen jeder Einflussgröße und deren jeweiligem Hygienewertanteil;

d. Ermittlung eines voraussichtlichen Ist-Hygienewerts am Ende der Reinigung aus den Hygienewertanteilen;

e. Vergleich des voraussichtlichen Ist-Hygienewerts mit dem Soll-Hygienewert; und

f. Beeinflussung mindestens einer Einflussgröße in Abhängigkeit von dem Ergebnis des Vergleichs.

Ausführungsform 2: Verfahren nach der vorhergehenden Ausführungsform, wobei Verfahrensschritt e. weiterhin eine Erkennung umfasst, ob der voraussichtliche Ist-Hygienewert kleiner ist als der Soll-Hygienewert.

Ausführungsform 3: Verfahren nach einer der vorhergehenden Ausführungsformen, wobei der Hygienewertanteil eine Hygienisierungsrate umfasst.

Ausführungsform 4: Verfahren nach einer der vorhergehenden Ausführungsformen, wobei der Soll-Hygienewert ausgewählt ist aus der Gruppe bestehend aus: einem bei der Reinigung insgesamt zu erreichenden AO-Wert nach DIN EN ISO 15883; einem zu erreichenden Hygienelevel nach DIN 10510, bei den Sollvorgaben von Tank- und Boiler Temperaturen nach DIN 10510; einer bei der Reinigung insgesamt zu erreichenden Anzahl an HUE-Einheiten nach dem NSF3-Standard; einer bei der Reinigung insgesamt zu erreichenden Reduktion einer Population eines Zielkeims, insbesondere einer bei der Reinigung insgesamt zu erreichenden Reduktion der Population von Staphylococcus aureus.

Ausführungsform 5: Verfahren nach der vorhergehenden Ausführungsform, wobei der Soll-Hygienewert einem Absterben einer Population von Staphylococcus aureus um 99,999% entspricht.

Ausführungsform 6: Verfahren nach einer der vorhergehenden Ausführungsformen, wobei die Einflussgrößen ausgewählt sind aus der Gruppe bestehend aus: einer Dauer der Reinigung; mindestens einer Temperatur des mindestens einen Reinigungsfluids; mindestens einer eine mechanische Einwirkung des mindestens einen Reinigungsfluids auf das Reinigungsgut charakterisierenden Messgröße, insbesondere einem Druck und/oder einer Flussrate des mindestens einen Reinigungsfluids; mindestens einer Konzentration mindestens eines Desinfektionsmittels in dem mindestens einen Reinigungsfluid; mindestens einer Konzentration mindestens eines Reinigerkonzentrats in dem mindestens einen Reinigungsfluid; mindestens einem pH-Wert des mindestens einen Reinigungsfluids; mindestens einer eine Wirkung einer Klarspülung charakterisierenden Messgröße.

Ausführungsform 7: Verfahren nach einer der vorhergehenden Ausführungsformen, wobei die mindestens zwei Einflussgrößen als erste Einflussgröße mindestens eine Temperatur des mindestens einen Reinigungsfluids umfassen sowie weiterhin mindestens eine weitere Einflussgröße.

Ausführungsform 8: Verfahren nach einer der vorhergehenden Ausführungsformen, wobei die zeitaufgelöste Erfassung der mindestens zwei Einflussgrößen zu vorgegebenen oder vorgebbaren Zeiten oder in vorgegebenen oder vorgebbaren Abständen erfolgt.

Ausführungsform 9: Verfahren nach einer der vorhergehenden Ausführungsformen, wobei die Beeinflussung der mindestens einen Einflussgröße zu vorgegebenen oder vorgebbaren Zeiten erfolgt.

Ausführungsform 10: Verfahren nach einer der vorhergehenden Ausführungsformen, wobei der Zusammenhang zwischen den Einflussgrößen und deren jeweiligen Hygienewertanteilen vorgegeben ist durch mindestens eine der folgenden Vorgaben: mindestens einer Kalibrationskurve, insbesondere mindestens einer Kurve zu einer Hygienewirkung, wobei die Kalibrationskurve jeweils einen Zusammenhang zwischen dem jeweiligen Hygienewertanteil der Einflussgröße und einer weiteren Einflussgröße aufzeigt, wobei die mindestens eine weitere Einflussgröße konstant gehalten wird; mindestens einer Kurvenschar mit einer Mehrzahl von Kalibrationskurven, wobei die Kalibrationskurven jeweils einen Zusammenhang zwischen dem jeweiligen Hygienewertanteil der Einflussgröße und einer weiteren Einflussgröße aufzeigen, wobei innerhalb jeder Kalibrationskurve der Kurvenschar die mindestens eine weitere Einflussgröße konstant gehalten wird, wobei sich die mindestens eine weitere Einflussgröße zwischen den Kalibrationskurven der Kurvenschar unterscheidet; mindestens einer Kalibrationstabelle, wobei die Kalibrationstabelle jeweils einen Zusammenhang zwischen dem jeweiligen Hygienewertanteil der Einflussgröße und einer weiteren Einflussgröße aufzeigt, wobei die mindestens eine weitere Einflussgröße konstant gehalten wird; mindestens einer Tabellenschar an Kalibrationstabellen mit einer Mehrzahl von Kalibrationstabellen, wobei die Kalibrationstabellen jeweils einen Zusammenhang zwischen dem jeweiligen Hygienewertanteil der Einflussgröße und einer weiteren Einflussgröße aufzeigen, wobei innerhalb jeder Kalibrationstabelle der Tabellenschar die mindestens eine weitere Einflussgröße konstant gehalten wird, wobei sich die mindestens eine weitere Einflussgröße zwischen den Kalibrationstabellen der Tabellenschar unterscheidet.

Ausführungsform 11: Verfahren nach einer der vorhergehenden Ausführungsformen, wobei das Verfahren mindestens eine Kalibration umfasst, wobei bei der Kalibration der Zusammenhang zwischen jeder Einflussgröße und deren jeweiligem Hygienewertanteil bestimmt wird.

Ausführungsform 12: Verfahren nach der vorhergehenden Ausführungsform, wobei der Zusammenhang nach der Kalibration in mindestens einem Datenspeicher gespeichert wird.

Ausführungsform 13: Verfahren nach einer der beiden vorhergehenden Ausführungsformen, wobei bei der Kalibration bestimmt wird, wie lange unter jeweiligen konstanten Bedingungen, einschließlich vorgegebener konstanter Werte der Einflussgrößen, benötigt wird, bis der vorgegebene Soll-Hygienewert erreicht wird, insbesondere ein vorgegebener Reduktionsfaktor oder eine vorgegebene Reduktion mindestens eines Zielkeims, wobei aus dieser Information über die Zeitdauer der Hygienewertanteil bestimmt wird, unter Berücksichtigung eines anfänglichen Hygienewerts, des Soll-Hygienewerts und der Zeitdauer.

Ausführungsform 14: Verfahren nach einer der vorhergehenden Ausführungsformen, wobei der voraussichtliche Ist-Hygienewert am Ende der Reinigung aus den Hygienewertanteilen bestimmt wird unter der Annahme, dass die aktuellen Einflussgrößen für den Rest der Reinigung unverändert bleiben.

Ausführungsform 15: Verfahren nach einer der vorhergehenden Ausführungsformen, wobei der voraussichtliche Ist-Hygienewert am Ende der Reinigung aus den Hygienewertanteilen berechnet wird unter Verwendung mindestens eines Algorithmus, ausgewählt aus der Gruppe bestehend aus: einer Integration der Summe der Hygienewertanteile der Einflussgrößen über die voraussichtliche restliche Dauer der Reinigung; einer Gesamtheit der zeitlichen Riemann-Summen der jeweiligen Hygienewertanteile der Einflussgrößen über die voraussichtliche restliche Dauer der Reinigung.

Ausführungsform 16: Verfahren nach einer der vorhergehenden Ausführungsformen, wobei die Dauer der Reinigung verändert wird, wenn bei dem Vergleich erkannt wird, dass der voraussichtliche Ist-Hygienewert von dem Soll-Hygienewert abweicht.

Ausführungsform 17: Verfahren nach der vorhergehenden Ausführungsform, wobei die Dauer der Reinigung erhöht wird, wenn erkannt wird, dass der voraussichtliche Ist-Hygienewert kleiner ist als der Soll-Hygienewert.

Ausführungsform 18: Verfahren nach einer der beiden vorhergehenden Ausführungsformen, wobei die Dauer der Reinigung reduziert wird, wenn erkannt wird, dass der voraussichtliche Ist-Hygienewert größer ist als der Soll-Hygienewert.

Ausführungsform 19: Verfahren nach einer der drei vorhergehenden Ausführungsformen, wobei mindestens eine Maßnahme zur Veränderung der Dauer der Reinigung eingesetzt wird, ausgewählt aus der Gruppe bestehend aus: mindestens eine Programmdauer mindestens eines Reinigungsprogramms wird verändert; mindestens eine Transportgeschwindigkeit mindestens einer Transportvorrichtung, mittels derer das Reinigungsgut durch die mindestens eine Reinigungskammer transportiert wird, wird verändert.

Ausführungsform 20: Verfahren nach einer der vorhergehenden Ausführungsformen, wobei mindestens eine Konzentration mindestens eines Zusatzstoffs in dem mindestens einen Reinigungsfluid verändert wird, wenn erkannt wird, dass der voraussichtliche Ist-Hygienewert von dem Soll-Hygienewert abweicht.

Ausführungsform 21: Verfahren nach der vorhergehenden Ausführungsform, wobei die mindestens eine Konzentration des mindestens einen Zusatzstoffs in dem mindestens einen Reinigungsfluid erhöht wird, wenn erkannt wird, dass der voraussichtliche Ist-Hygienewert den Soll-Hygienewert unterschreitet.

Ausführungsform 22: Verfahren nach einer der beiden vorhergehenden Ausführungsformen, wobei der Zusatzstoff mindestens einen Stoff umfasst, ausgewählt aus der Gruppe bestehend aus einem Reinigerkonzentrat und einem Desinfektionsmittel, insbesondere Chlor.

Ausführungsform 23: Verfahren nach einer der vorhergehenden Ausführungsformen, wobei mindestens eine mechanische Beaufschlagung des Reinigungsguts mit dem mindestens einen Reinigungsfluid verändert wird, wenn bei dem Vergleich erkannt wird, dass der voraussichtliche Ist-Hygienewert von dem Soll-Hygienewert abweicht.

Ausführungsform 24: Verfahren nach der vorhergehenden Ausführungsform, wobei eine Intensität der mechanischen Beaufschlagung erhöht wird, wenn erkannt wird, dass der voraussichtliche Ist-Hygienewert kleiner ist als der Soll-Hygienewert.

Ausführungsform 25: Verfahren nach einer der beiden vorhergehenden Ausführungsformen, wobei eine Intensität der mechanischen Beaufschlagung erniedrigt wird, wenn erkannt wird, dass der voraussichtliche Ist-Hygienewert größer ist als der Soll-Hygienewert.

Ausführungsform 26: Verfahren nach einer der drei vorhergehenden Ausführungsformen, wobei mindestens eine Maßnahme zur Veränderung der Intensität der mechanischen Beaufschlagung eingesetzt wird, ausgewählt aus der Gruppe bestehend aus: mindestens ein Druck des mindestens einen Reinigungsfluids wird verändert; mindestens eine Flussrate des mindestens einen Reinigungsfluids wird verändert.

Ausführungsform 27: Verfahren nach einer der vorhergehenden Ausführungsformen, wobei in f. zur Beeinflussung der mindestens einen Einflussgröße eine Maßnahme zur Beeinflussung eingesetzt wird, wobei die Maßnahme zur Beeinflussung in Abhängigkeit von einer vorgegebenen und/oder vorgebbaren Maßnahmenhierarchie erfolgt.

Ausführungsform 28: Verfahren nach einer der vorhergehenden Ausführungsformen, wobei das Reinigungsgut mittels mindestens einer Transportvorrichtung durch die mindestens eine Reinigungskammer transportiert wird.

Ausführungsform 29: Verfahren nach der vorhergehenden Ausführungsform, wobei das Reinigungsgut eine Mehrzahl an zu reinigenden Gütern umfasst, wobei jedem Gut ein Hygienisierungskonto zugewiesen wird, auf welchem bisherige Hygienisierungseinheiten, denen das Gut unterworfen wurde, kumuliert gespeichert werden.

Ausführungsform 30: Verfahren nach einer der beiden vorhergehenden Ausführungsformen, wobei das Reinigungsgut eine Mehrzahl an zu reinigenden Gütern umfasst, wobei die Transportvorrichtung in Abschnitte unterteilt ist, wobei jedem Abschnitt ein Hygienisierungskonto zugewiesen wird, auf welchem bisherige Hygienisierungseinheiten, denen in dem jeweiligen Abschnitt aufgenommenes Gut unterworfen wurde, kumuliert gespeichert werden.

Ausführungsform 31: Verfahren nach einer der beiden vorhergehenden Ausführungsformen, wobei das Hygienisierungskonto wieder auf Null gesetzt wird, wenn ein Fehler in dem Reinigungsvorgang festgestellt wird.

Ausführungsform 32: Verfahren nach einer der vier vorhergehenden Ausführungsformen, wobei die Reinigungskammer in eine Mehrzahl von Reinigungszonen unterteilt ist, wobei jede Reinigungszone mindestens eine Beaufschlagungsvorrichtung aufweist, wobei das Reinigungsgut mittels der Transportvorrichtung nacheinander durch die Reinigungszonen transportiert wird.

Ausführungsform 33: Verfahren nach der vorhergehenden Ausführungsform, wobei das Verfahren mindestens einen Überprüfungsschritt umfasst, wobei der Überprüfungsschritt durchgeführt wird, solange sich das Reinigungsgut innerhalb der letzten Reinigungszone in einer Transportrichtung der Transportvorrichtung befindet, wobei bei dem Überprüfungsschritt der voraussichtliche Ist-Hygienewert nochmals mit dem Soll-Hygienewert verglichen wird und wobei bei einem Unterschreiten des Soll-Hygienewerts mindestens eine Sicherungsmaßnahme ergriffen wird.

Ausführungsform 34: Verfahren nach der vorhergehenden Ausführungsform, wobei die Sicherungsmaßnahme mindestens eine Maßnahme umfasst, ausgewählt aus der Gruppe bestehend aus: die Transportvorrichtung wird angehalten; die Transportvorrichtung wird verlangsamt; ein Druck des Reinigungsfluids in der Beaufschlagungsvorrichtung der letzten Reinigungszone wird erhöht; eine Flussrate des Reinigungsfluids in der Beaufschlagungsvorrichtung der letzten Reinigungszone wird erhöht.

Ausführungsform 35: Verfahren nach einer der beiden vorhergehenden Ausführungsformen, wobei die letzte Reinigungszone eine Klarspülzone ist, insbesondere eine Frischwasser-Klarspülzone.

Ausführungsform 36: Reinigungsvorrichtung zum Reinigen von Reinigungsgut, umfassend mindestens eine Reinigungskammer und mindestens eine Beaufschlagungsvorrichtung zum Beaufschlagen des Reinigungsguts in der Reinigungskammer mit mindestens einem Reinigungsfluid, wobei die Reinigungsvorrichtung weiterhin umfasst:

I. mindestens einen Datenspeicher zur Speicherung einer Vorgabe eines bei der Reinigung zu erreichenden Soll-Hygienewerts;

II. mindestens zwei Messvorrichtungen zur zeitaufgelösten Erfassung mindestens zweier Einflussgrößen, welche einen Einfluss auf eine Hygienisierung des Reinigungsguts haben;

III. mindestens eine Bestimmungsvorrichtung zur Bestimmung von Hygienewertanteilen aus den Einflussgrößen unter Verwendung eines vorgegebenen Zusammenhangs zwischen jeder Einflussgröße und deren jeweiligem Hygienewertanteil;

IV. mindestens eine Auswertevorrichtung zur Ermittlung eines voraussichtlichen Ist-Hygienewerts an einem Ende der Reinigung aus den Hygienewertanteilen;

V. mindestens eine Vergleichsvorrichtung zum Vergleich des voraussichtlichen Ist-Hygienewerts mit dem Soll-Hygienewert; und

VI. mindestens eine Beeinflussungsvorrichtung zur Beeinflussung mindestens einer Einflussgröße in Abhängigkeit von dem Ergebnis des Vergleichs.

Ausführungsform 37: Reinigungsvorrichtung nach der vorhergehenden Ausführungsform, wobei die Reinigungsvorrichtung eingerichtet ist, um ein Verfahren nach einer der vorhergehenden Ausführungsformen durchzuführen.

Ausführungsform 38: Reinigungsvorrichtung nach einer der vorhergehenden, eine Reinigungsvorrichtung betreffenden Ausführungsformen, wobei die Reinigungsvorrichtung mindestens eine Steuerung aufweist, wobei der Datenspeicher, die Bestimmungsvorrichtung, die Auswertevorrichtung, die Vergleichsvorrichtung und die Beeinflussungsvorrichtung Bestandteile der Steuerung sind.

Ausführungsform 39: Reinigungsvorrichtung nach der vorhergehenden Ausführungsform, wobei die Steuerung mindestens eine Datenverarbeitungsvorrichtung, insbesondere mindestens einen Prozessor, umfasst.

Ausführungsform 40: Reinigungsvorrichtung nach der vorhergehenden Ausführungsform, wobei die Datenverarbeitungsvorrichtung programmtechnisch eingerichtet ist, um den Einflussgrößen aufgrund des vorgegebenen Zusammenhangs jeweils einen Hygienewertanteil zuzuweisen und um den voraussichtlichen Ist-Hygienewert am Ende der Reinigung aus den Hygienewertanteilen zu bestimmen.

Ausführungsform 41: Reinigungsvorrichtung nach der vorhergehenden Ausführungsform, wobei die Datenverarbeitungsvorrichtung weiterhin programmtechnisch eingerichtet ist, um den voraussichtlichen Ist-Hygienewert mit dem Soll-Hygienewert zu vergleichen und um zu erkennen, ob der voraussichtliche Ist-Hygienewert kleiner ist als der Soll-Hygienewert.

Ausführungsform 42: Reinigungsvorrichtung nach der vorhergehenden Ausführungsform, wobei die Datenverarbeitungsvorrichtung weiterhin programmtechnisch eingerichtet ist, um die mindestens eine Einflussgröße in Abhängigkeit von dem Ergebnis des Vergleichs zu beeinflussen.

Ausführungsform 43: Reinigungsvorrichtung nach einer der vorhergehenden, eine Reinigungsvorrichtung betreffenden Ausführungsformen, wobei der Datenspeicher mindestens einen Speicher umfasst, ausgewählt aus einem flüchtigen Datenspeicher und einem nicht flüchtigen Datenspeicher.

Ausführungsform 44: Reinigungsvorrichtung nach einer der vorhergehenden, eine Reinigungsvorrichtung betreffenden Ausführungsformen, wobei die Messvorrichtungen mindestens zwei Sensoren umfassen, ausgewählt aus der Gruppe bestehend aus: mindestens einem Temperatursensor zur Erfassung mindestens einer Temperatur des mindestens einen Reinigungsfluids; mindestens einem mechanischen oder fluidischen Sensor zur Erfassung mindestens einer Messgröße einer mechanischen Einwirkung des mindestens einen Reinigungsfluids auf das Reinigungsgut, insbesondere mindestens einen Drucksensor zur Erfassung mindestens eines Drucks des mindestens einen Reinigungsfluids und/oder mindestens einen Flusssensor zur Erfassung mindestens eines Flusses des mindestens einen Reinigungsfluids; mindestens einem Konzentrationssensor zur Erfassung mindestens einer Konzentration mindestens eines Desinfektionsmittels in dem mindestens einem Reinigungsfluid; mindestens einem Konzentrationssensor zur Erfassung mindestens einer Konzentration mindestens eines Reinigerkonzentrats in dem mindestens einem Reinigungsfluid; mindestens einem pH-Sensor zur Erfassung mindestens eines pH-Werts des mindestens einen Reinigungsfluids. Ausführungsform 45: Reinigungsvorrichtung nach einer der vorhergehenden, eine Reinigungsvorrichtung betreffenden Ausführungsformen, wobei die Reinigungsvorrichtung ausgewählt ist aus der Gruppe bestehend aus einer Transportspülmaschine und einer Programmspülmaschine.

Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigen:

Figur 1      ein Ausführungsbeispiel eines erfindungsgemäßen Verfahrens;

Figur 2      ein Ausführungsbeispiel einer erfindungsgemäßen Reinigungsvorrichtung;

Figur 3      einen Vergleich der erreichten Hygienewertanteile pro Sekunde nach A0-Verfahren, nach HUE- Verfahren und nach SUE-Verfahren; und

Figur 4      einen vorgegebenen Zusammenhang zwischen der Einflussgröße Temperatur, deren Hygienewertanteil nach dem SUE-Verfahren und der Konzentration.

Beschreibung der Ausführungsbeispiele

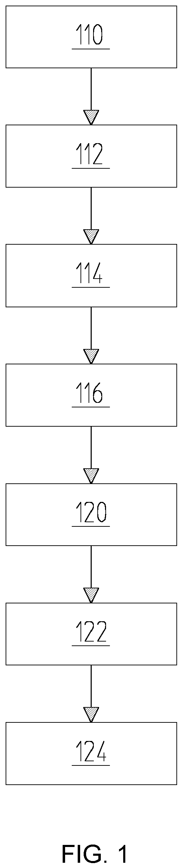

In Figur 1 ist ein Ausführungsbeispiel eines erfindungsgemäßen Verfahrens zum Reinigen von Reinigungsgut 143 schematisch dargestellt. Das Verfahren umfasst eine Vorgabe eines bei der Reinigung zu erreichenden Soll-Hygienewerts 110. Der Soll-Hygienewert kann ausgewählt sein aus der Gruppe bestehend aus: einem bei der Reinigung insgesamt zu erreichenden AO-Wert nach DIN EN ISO 15883; einem zu erreichenden Hygienelevel nach DIN 10510, bei den Sollvorgaben von Tank- und Boiler Temperaturen nach DIN 10510; einer bei der Reinigung insgesamt zu erreichenden Anzahl an HUE-Einheiten nach dem NSF3-Standard; einer bei der Reinigung insgesamt zu erreichenden Reduktion einer Population eines Zielkeims, insbesondere einer bei der Reinigung insgesamt zu erreichenden Reduktion der Population von Staphylococcus aureus. Der Soll-Hygienewert kann einem Absterben einer Population von Staphylococcus aureus um 99,999% entsprechen. Bevorzugt kann der Hygienewert ein SUE-Wert sein. Der SUE-Wert pro Sekunde kann ein Maß für die Reduktion des Zielkeims sein, welches die Abhängigkeit von der Temperatur und der verwendeten Reinigerchemie berücksichtigt, wobei

$$SUE/sec = Ae^{BT},$$

wobei A = 1/7000000 und B = 0,191. Ein SUE-Wert von 100 entspricht dabei einer 5 log-Stufen Reduktion des Zielkeims. Die Parameter A und B sind experimentelle Werte, bestimmt unter Verwendung einer Population von Staphylococcus aureus als Zielkeimpopulation. Beispielsweise kann die Vorgabe des bei der Reinigung zu erreichenden Soll-Hygienewerts 110 eine Auswahl eines Soll-Hygienewerts aus einer Liste und/oder eine Eingabe eines Soll-Hygienewerts, beispielsweise in eine Mensch-Maschine-Schnittstelle der Reinigungsvorrichtung 126, insbesondere einer Steuerung 118 der Reinigungsvorrichtung 126, umfassen.

Das Verfahren umfasst eine zeitaufgelöste Erfassung mindestens zweier Einflussgrößen 112, welche einen Einfluss auf eine Hygienisierung des Reinigungsguts 143 haben. Die Einflussgrößen können ausgewählt sein aus der Gruppe bestehend aus: einer Dauer der Reinigung; mindestens einer Temperatur des mindestens einen Reinigungsfluids; mindestens einer eine mechanische Einwirkung des mindestens einen Reinigungsfluids auf das Reinigungsgut 143 charakterisierenden Messgröße, insbesondere einem Druck und/oder einer Flussrate des mindestens einen Reinigungsfluids; mindestens einer Konzentration mindestens eines Desinfektionsmittels in dem mindestens einen Reinigungsfluid; mindestens einer Konzentration mindestens eines Reinigerkonzentrats in dem mindestens einen Reinigungsfluid; mindestens einem pH-Wert des mindestens einen Reinigungsfluids; mindestens einer eine Wirkung einer Klarspülung charakterisierenden Messgröße. Die Einflussgrößen können sich gegenseitig beeinflussen. Eine Verminderung einer dieser Einflussgrößen kann jeweils durch eine Erhöhung einer oder mehrerer der anderen Einflussgrößen kompensiert werden. Die mindestens zwei Einflussgrößen können als erste Einflussgröße mindestens eine Temperatur des mindestens einen Reinigungsfluids sowie weiterhin mindestens eine weitere Einflussgröße umfassen.

Das Verfahren umfasst ein Bestimmen von Hygienewertanteilen aus den Einflussgrößen 114 unter Verwendung eines vorgegebenen Zusammenhangs zwischen jeder Einflussgröße und deren jeweiligem Hygienewertanteil. Der Zusammenhang zwischen den Einflussgrößen und deren jeweiligen Hygienewertanteilen kann vorgegeben sein durch mindestens eine der folgenden Vorgaben: mindestens einer Kalibrationskurve, insbesondere mindestens einer Kurve zu einer Hygienewirkung, wobei die Kalibrationskurve jeweils einen Zusammenhang zwischen dem jeweiligen Hygienewertanteil der Einflussgröße und einer weiteren Einflussgröße aufzeigt, wobei die mindestens eine weitere Einflussgröße konstant gehalten wird; mindestens einer Kurvenschar mit einer Mehrzahl von Kalibrationskurven, wobei die Kalibrationskurven jeweils einen Zusammenhang zwischen dem jeweiligen Hygienewertanteil der Einflussgröße und einer weiteren Einflussgröße aufzeigen, wobei innerhalb jeder Kalibrationskurve der Kurvenschar die mindestens eine weitere Einflussgröße konstant gehalten wird, wobei sich die mindestens eine weitere Einflussgröße zwischen den Kalibrationskurven der Kurvenschar unterscheidet; mindestens einer Kalibrationstabelle, wobei die Kalibrationstabelle jeweils einen Zusammenhang zwischen dem jeweiligen Hygienewertanteil der Einflussgröße und einer weiteren Einflussgröße aufzeigt, wobei die mindestens eine weitere Einflussgröße konstant gehalten wird; mindestens einer Tabellenschar an Kalibrationstabellen mit einer Mehrzahl von Kalibrationstabellen, wobei die Kalibrationstabellen jeweils einen Zusammenhang zwischen dem jeweiligen Hygienewertanteil der Einflussgröße und einer weiteren Einflussgröße aufzeigen, wobei innerhalb jeder Kalibrationstabelle der Tabellenschar die mindestens eine weitere Einflussgröße konstant gehalten wird, wobei sich die mindestens eine weitere Einflussgröße zwischen den Kalibrationstabellen der Tabellenschar unterscheidet. Auf diese Weise kann eine Abhängigkeit der einzelnen Einflussgrößen zueinander bestimmt werden. Bei einer Berücksichtigung mindestens zweier Einflussgrößen, insbesondere aller Einflussgrößen, kann ein mehrdimensionales Feld bestimmt werden, in welchem die einzelnen Einflussgrößen variiert werden können.

Das Verfahren kann mindestens eine Kalibration 116 umfassen, in welchem der Zusammenhang zwischen jeder Einflussgröße und deren jeweiligem Hygienewertanteil bestimmt werden kann. Beispielsweise kann die Kalibration 116 einmalig, beispielsweise bei einer ersten Inbetriebnahme erfolgen. Beispielsweise kann die Kalibration 116 wiederholt erfolgen, beispielsweise in vorgebbaren oder wählbaren Zeitintervallen. Der Zusammenhang kann nach der Kalibration 116 in mindestens einem Datenspeicher, beispielsweise einer Steuerung 118, gespeichert werden. Beispielsweise kann der Zusammenhang in einer Tabelle gespeichert werden, wobei beispielsweise die Kalibrationskurven als diskrete Werte gespeichert werden. Bei der Kalibration 116 kann bestimmt werden, wie lange unter jeweiligen konstanten Bedingungen, einschließlich vorgegebener konstanter Werte der Einflussgrößen, benötigt wird, bis der vorgegebene Soll-Hygienewert erreicht wird, insbesondere ein vorgegebener Reduktionsfaktor oder eine vorgegebene Reduktion mindestens eines Zielkeims, wobei aus dieser Information über die Zeitdauer der Hygienewertanteil bestimmt wird, unter Berücksichtigung eines anfänglichen Hygienewerts, des Soll-Hygienewerts und der Zeitdauer.

Das Verfahren umfasst eine Ermittlung eines voraussichtlichen Ist-Hygienewerts am Ende der Reinigung aus den Hygienewertanteilen 120. Die Ermittlung des voraussichtlichen Ist-Hygienewertes 120 kann eine Berücksichtigung und/oder ein Aufsummieren aller bisher, also bis zum Zeitpunkt der Ermittlung des voraussichtlichen Ist-Hygienewerts 120, erreichten Hygienewertanteile umfassen. Insbesondere können alle seit einem Startzeitpunkt eines Reinigungsprogramms auf das Reinigungsgut 143 wirkende Hygienewertanteile berücksichtigt und/oder aufsummiert werden. Der voraussichtliche Ist-Hygienewert kann eine Prognose für den Ist-Hygienewert am Ende der Reinigung sein. Der voraussichtliche Ist-Hygienewert kann am Ende der Reinigung aus den Hygienewertanteilen bestimmt werden unter der Annahme, dass die aktuellen Einflussgrößen für den Rest der Reinigung unverändert bleiben. Der voraussichtliche Ist-Hygienewert am Ende der Reinigung kann aus den Hygienewertanteilen berechnet werden unter Verwendung mindestens eines Algorithmus, ausgewählt aus der Gruppe bestehend aus: einer Integration der Summe der Hygienewertanteile der Einflussgrößen über die voraussichtliche restliche Dauer der Reinigung; einer Gesamtheit der zeitlichen Riemann-Summen der jeweiligen Hygienewertanteile der Einflussgrößen über die voraussichtliche restliche Dauer der Reinigung.

Das Verfahren umfasst einen Vergleich des voraussichtlichen Ist-Hygienewerts mit dem Soll-Hygienewert 122. Der Vergleich 122 kann ein Bestimmen eines Überschreitens und/oder Unterschreitens des voraussichtlichen Ist-Hygienewerts und des Soll-Hygienewerts umfassen. Der Vergleich 122 kann einen mathematischen Vergleich umfassen. Insbesondere kann der Vergleich 122 eine Erkennung umfassen, ob der voraussichtliche Ist-Hygienewert kleiner ist als der Soll-Hygienewert. Das Verfahren umfasst eine Beeinflussung mindestens einer Einflussgröße 124 in Abhängigkeit von dem Ergebnis des Vergleichs. Die Beeinflussung der mindestens einen Einflussgröße 124 kann zu vorgegebenen oder vorgebbaren Zeiten erfolgen.

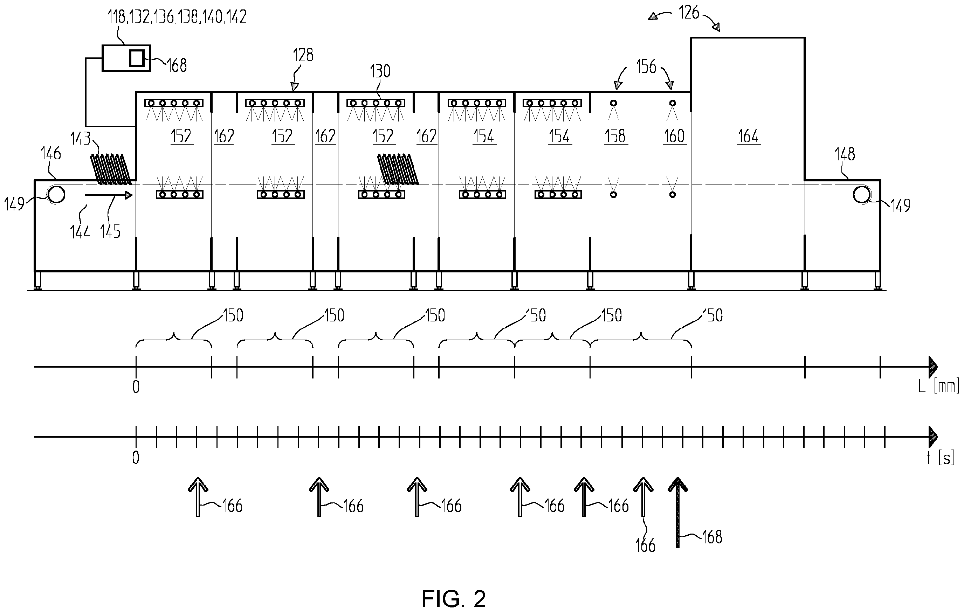

Figur 2 zeigt eine schematische Darstellung einer Ausführungsform einer Reinigungsvorrichtung 126 mit mindestens einer Reinigungskammer 128 und mindestens einer Beaufschlagungsvorrichtung 130 zum Beaufschlagen des Reinigungsguts 143 in der Reinigungskammer 128 mit mindestens einem Reinigungsfluid. Die Reinigungsvorrichtung 126 umfasst mindestens einen Datenspeicher 132 zur Speicherung einer Vorgabe eines bei der Reinigung zu erreichenden Soll-Hygienewerts, mindestens zwei, in Figur 2 nicht dargestellten, Messvorrichtungen 134 zur zeitaufgelösten Erfassung mindestens zweier Einflussgrößen, welche einen Einfluss auf eine Hygienisierung des Reinigungsguts 143 haben, mindestens eine Bestimmungsvorrichtung 136 zur Bestimmung von Hygienewertanteilen aus den Einflussgrößen unter Verwendung eines vorgegebenen Zusammenhangs zwischen jeder Einflussgröße und deren jeweiligem Hygienewertanteil, mindestens eine Auswertevorrichtung 138 zur Ermittlung eines voraussichtlichen Ist-Hygienewerts an einem Ende der Reinigung aus den Hygienewertanteilen, mindestens eine Vergleichsvorrichtung 140 zum Vergleich des voraussichtlichen Ist-Hygienewerts mit dem Soll-Hygienewert, und mindestens eine Beeinflussungsvorrichtung 142 zur Beeinflussung mindestens einer Einflussgröße in Abhängigkeit von dem Ergebnis des Vergleichs. Der Datenspeicher 132, die Bestimmungsvorrichtung 136, die Auswertevorrichtung 138, die Vergleichsvorrichtung 140 und die Beeinflussungsvorrichtung 142 können Bestandteile der Steuerung 118 sein.

In dem in Figur 2 dargestellten Ausführungsbeispiel kann das Reinigungsgut 143, beispielsweise in Form von Geschirr, mittels mindestens einer Transportvorrichtung 144 in der Transportrichtung 145 durch die Reinigungskammer 128, beispielsweise in Form eines Tunnels befördert werden. Die Transportrichtung 145 ist in Figur 2 mit einem Pfeil gekennzeichnet. Zu diesem Zweck kann die Reinigungsvorrichtung 126 beispielsweise als Bandtransportmaschine oder als Korbtransportmaschine ausgestaltet sein. Beispielsweise kann mindestens ein Einlauf 146 vorgesehen sein, an welchem das Reinigungsgut 143 auf die Transportvorrichtung 144 aufgegeben wird, und mindestens ein Auslauf 148, an welchem das gereinigte Reinigungsgut 143 entnommen werden kann. Die Transportvorrichtung 144 kann dementsprechend beispielsweise ein Förderband umfassen. Auch andere Ausgestaltungen sind möglich. Die Transportvorrichtung 144 kann beispielsweise mindestens einen Antrieb 149 umfassen.

Die Reinigungskammer 128 kann in eine Mehrzahl von Reinigungszonen 150 unterteilt sein. In den Reinigungszonen 150 kann das Reinigungsgut 143 über die Beaufschlagungsvorrichtung 130, beispielsweise eine Fluidvorrichtungen, mit Reinigungsfluid beaufschlagt werden. Jede Reinigungszone 150 kann mindestens eine Beaufschlagungsvorrichtung 130 aufweisen. Beispielsweise kann zu diesem Zweck mindestens ein, in Figur 2 nicht dargestelltes, Düsensystem vorgesehen sein, welches, beispielsweise über eine in Figur 2 nicht dargestellte Pumpe und ein nicht dargestelltes Leitungssystem aus einem Tank mit Reinigungsfluid gespeist werden kann. Das Reinigungsgut 143 kann mittels der Transportvorrichtung 144 nacheinander durch die Reinigungszonen 150 transportiert werden. In Figur 2 ist der Transportweg L in mm und die Länge der Reinigungszonen 150 gezeigt.

In der in Figur 2 gezeigten Ausführungsform kann die Reinigungsvorrichtung 126 beispielsweise mehrere Waschtanks 152 aufweisen, in welchen das Reinigungsgut 143 mit mindestens einem Reinigungsfluid bei einer Temperatur beaufschlagt wird. In den Waschtanks 152, der sogenannten Spülzone, kann beispielsweise ein nicht dargestelltes Spülzonen-Düsensystem vorgesehen sein, welches, beispielsweise über eine ebenfalls nicht dargestellte Pumpe und ein ebenfalls nicht dargestelltes Leitungssystem, aus einem Spültank mit Reinigungsfluid gespeist werden kann. Weiter kann die Reinigungsvorrichtung 126 eine oder mehrere Thermodesinfektionszonen 154 aufweisen, welche eingerichtet sind, eine Thermo-Desinfektion durchzuführen. Die Reinigungsvorrichtung 126 kann mindestens eine Klarspülzone 156 aufweisen, welche beispielsweise in eine Pumpenklarspülung 158 und in eine Frischwasserklarspülung 160 unterteilt ist. In der Klarspülzone 158 und 160 kann beispielsweise ein Nachspüldüsensystem vorgesehen sein, welches mit erwärmtem Frischwasser und/oder mit Nachspülfluid aus einem Nachspültank gespeist werden kann. Die Pumpenklarspülung 158 kann eingerichtet sein, mindestens einen Klarspüldurchgang in einem Umwälzverfahren mit mindestens einer Klarspülflüssigkeit durchzuführen. In der Frischwasserklarspülung 160 kann Frischwasser, welches optional erwärmt und/oder mit einem Klarspüler versetzt sein kann, insbesondere als so genannte Klarspüllösung, über die Beaufschlagungsvorrichtung 130 auf das Reinigungsgut 143 aufgebracht werden und so letzte Schmutzreste und Reste des Reinigungsfluids vom Reinigungsgut 143 abgespült werden. Die Reinigungsvorrichtung 126 kann weiterhin eine oder mehrere Neutralzonen 162 aufweisen in welchen keine Reinigung erfolgt. Nach Durchlaufen der Reinigungszonen 150 kann das Reinigungsgut 143 dann in mindestens eine Trocknungszone 164 eintreten, in welcher das Reinigungsgut 143 beispielsweise über ein Gebläse mit Warmluft beaufschlagt werden kann, um die Trocknung des Reinigungsguts 143 zu beschleunigen.

Das Bestimmen von Hygienewertanteilen aus den Einflussgrößen unter Verwendung eines vorgegebenen Zusammenhangs zwischen jeder Einflussgröße und deren jeweiligem Hygienewertanteil 120 und ein Aufsummieren von bisher erreichten Hygienewertanteilen kann zu verschiedenen Zeitpunkten während des Transports des Reinigungsguts 143 durch die Reinigungskammer 128 erfolgen. In Figur 2 ist eine Zeitachse t mit Zeiteinheiten gezeigt. Beispielhafte Zeitpunkte, an welchen eine Bestimmung von Hygienewertanteilen 120 und Aufsummieren der bestimmten Hygienewertanteile erfolgen kann, sind in Figur 2 als Pfeile mit Bezugszahl 166 gekennzeichnet. Insbesondere kann an diesen Zeitpunkten 166 eine Anzeige der bisher erreichten Hygienewerte erfolgen, beispielsweise in einer Anzeigevorrichtung 168, insbesondere einem Display, der Steuerung 118. Die Bestimmungsvorrichtung 136 ist eingerichtet, Hygienewertanteile aus den Einflussgrößen unter Verwendung des vorgegebenen Zusammenhangs zwischen jeder Einflussgröße und deren jeweiligem Hygienewertanteil zu bestimmen. Zur Erfassung der Einflussgröße weist die Reinigungsvorrichtung 126 mindestens zwei, in Figur 2 nicht dargestellte, Messvorrichtungen 134 auf, welche eingerichtet sind, mindestens eine Einflussgröße zeitaufgelöst zu erfassen. Die Messvorrichtungen 134 können mindestens zwei Sensoren umfassen, ausgewählt aus der Gruppe bestehend aus: mindestens einem Temperatursensor zur Erfassung mindestens einer Temperatur des mindestens einen Reinigungsfluids; mindestens einem mechanischen oder fluidischen Sensor zur Erfassung mindestens einer Messgröße einer mechanischen Einwirkung des mindestens einen Reinigungsfluids auf das Reinigungsgut 143, insbesondere mindestens einen Drucksensor zur Erfassung mindestens eines Drucks des mindestens einen Reinigungsfluids und/oder mindestens einen Flusssensor zur Erfassung mindestens eines Flusses des mindestens einen Reinigungsfluids; mindestens einem Konzentrationssensor zur Erfassung mindestens einer Konzentration mindestens eines Desinfektionsmittels in dem mindestens einen Reinigungsfluid; mindestens einem Konzentrationssensor zur Erfassung mindestens einer Konzentration mindestens eines Reinigerkonzentrats in dem mindestens einen Reinigungsfluid; mindestens einem pH-Sensor zur Erfassung mindestens eines pH-Werts des mindestens einen Reinigungsfluids.

An den Zeitpunkten 166 kann der voraussichtliche Ist-Hygienewert mit dem Soll-Hygienewert verglichen werden und bei einem Unterschreiten des Soll-Hygienewerts die Beeinflussung mindestens einer Einflussgröße erfolgen. Beispielsweise kann eine Dauer der Reinigung und/oder eine mechanische Wirkung und/oder eine Wirkung der Klarspülung und/oder eine Konzentration und/oder Dosierung und/oder mindestens ein pH-Wert und/oder die Temperatur des mindestens einen Reinigungsfluids in mindestens einer in Transportrichtung nachfolgenden Reinigungszone erhöht werden.

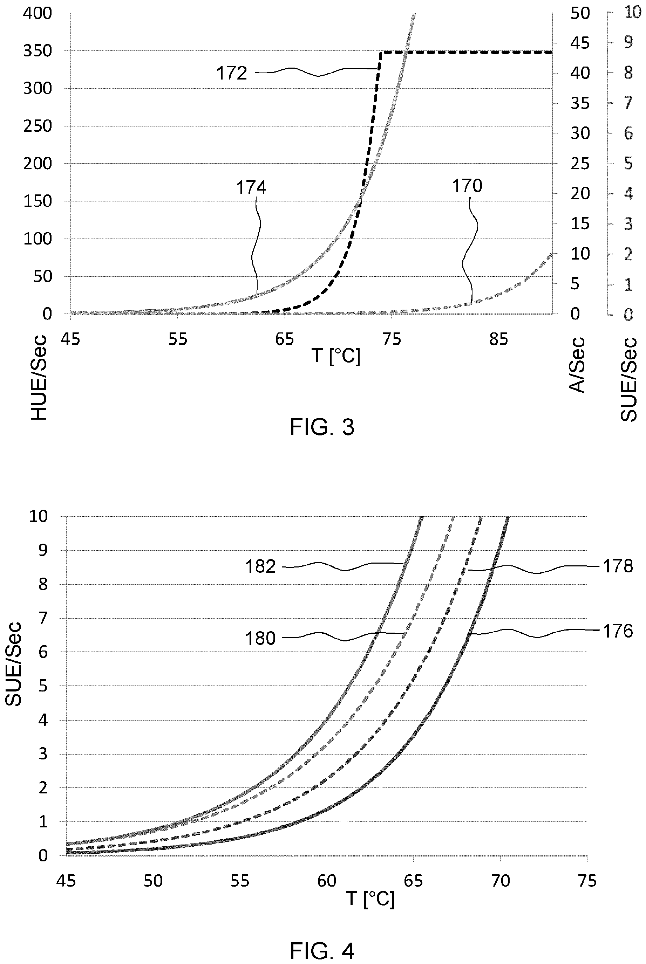

Die Reinigungsvorrichtung 126 kann eingerichtet sein, mindestens einen Überprüfungsschritt durchzuführen. Der Überprüfungsschritt kann durchgeführt werden, solange sich das Reinigungsgut 143 innerhalb einer letzten Reinigungszone, beispielsweise der Klarspülzone 156, insbesondere der Frischwasserklarspülzone 160, in einer Transportrichtung befindet. Beispielsweise kann der Überprüfungsschritt zu einem Zeitpunkt gekennzeichnet mit Pfeil und Bezugsziffer 168 erfolgen. Bei dem Überprüfungsschritt kann der voraussichtliche Ist-Hygienewert nochmals mit dem Soll-Hygienewert verglichen werden und bei einem Unterschreiten des Soll-Hygienewerts mindestens eine Sicherungsmaßnahme ergriffen werden. Die Sicherungsmaßnahme kann mindestens eine Maßnahme umfassen, ausgewählt aus der Gruppe bestehend aus: die Transportvorrichtung 145 wird angehalten; die Transportvorrichtung 145 wird verlangsamt; ein Druck des Reinigungsfluids in der Beaufschlagungsvorrichtung 130 der letzten Reinigungszone 160 wird erhöht; eine Flussrate des Reinigungsfluids in der Beaufschlagungsvorrichtung 130 der letzten Reinigungszone 160 wird erhöht. Figur 3 zeigt einen Vergleich der erreichten Hygienewertanteile pro Sekunde nach A0-Verfahren (gekennzeichnet mit Bezugsziffer 170), nach HUE-Verfahren (gekennzeichnet mit Bezugsziffer 172) und nach dem SUE-Verfahren (gekennzeichnet mit Bezugsziffer 174) für eine Konzentration (2,5 ml/l Tensid + 2,5 ml/l Kaliumhydroxid). Auf der linken Y-Achse ist der HUE-Wert pro Sekunde von 0 bis 400 und auf einer ersten rechten Y-Achse der A0-Wert pro Sekunde von 0 bis 50 und auf einer zweiten rechten Y-Achse der SUE pro Sekunde von 0 bis 10 aufgetragen. Alle Kurven zeigen eine starke Temperaturabhängigkeit. A0-Verfahren und HUE-Verfahren berücksichtigen jedoch nicht den Einfluss der Konzentration oder Zusammensetzung des mindestens einen Zusatzstoffes des Reinigungsfluids auf die Hygienewirkung. So können hohe Temperaturen notwendig sein, um ein nach A0- oder HUE Verfahren vorgegebenes Hygienelevel zu erreichen. Das SUE-Verfahren kann eine Berücksichtigung der Konzentration oder Zusammensetzung des mindestens einen Zusatzstoffes des Reinigungsfluids auf die Hygienewirkung erlauben und so bei gleichbleibender Hygienewirkung einen Betrieb der Reinigungsvorrichtung bei niedrigen Temperaturen ermöglichen.

Figur 4 zeigt einen vorgegebenen Zusammenhang zwischen der Einflussgröße Temperatur T in °C, deren Hygienewertanteil nach dem SUE-Verfahren und der Konzentration. Kurve 176 zeigt den Einfluss der Temperatur für eine Konzentration von (2,5 ml/l Tensid + 2,5 ml/l Kaliumhydroxid). Kurve 178 zeigt den Einfluss der Temperatur für eine Konzentration von (5 ml/l Tensid + 5 ml/l Kaliumhydroxid). Kurve 180 zeigt den Einfluss der Temperatur für eine Konzentration von (7,5 ml/l Tensid + 7,5 ml/l Kaliumhydroxid). Kurve 182 zeigt den Einfluss der Temperatur für eine Konzentration von (10 ml/l Tensid + 10 ml/l Kaliumhydroxid). Durch Erhöhung der Konzentration kann bei gleichbleibender Temperatur ein höherer Hygienisierungswertanteil erreicht werden. Eine Erhöhung der Konzentration kann somit einen Temperaturabfall kompensieren.

Bezugszeichenliste

| | |
|---|---|
| 110 | Vorgabe eines bei der Reinigung zu erreichenden Soll-Hygienewerts |
| 112 | zeitaufgelöste Erfassung mindestens zweier Einflussgrößen |
| 114 | Bestimmen von Hygienewertanteilen aus den Einflussgrößen |
| 116 | Kalibration |
| 118 | Steuerung |
| 120 | Ermittlung eines voraussichtlichen Ist-Hygienewerts am Ende der Reinigung aus den Hygienewertanteilen |
| 122 | Vergleich des voraussichtlichen Ist-Hygienewerts mit dem Soll-Hygienewert |
| 124 | Beeinflussung mindestens einer Einflussgröße |
| 126 | Reinigungsvorrichtung |
| 128 | Reinigungskammer |
| 130 | Beaufschlagungsvorrichtung |
| 132 | Datenspeicher |
| 134 | Messvorrichtungen |
| 136 | Bestimmungsvorrichtung |
| 138 | Auswertevorrichtung |
| 140 | Vergleichsvorrichtung |
| 142 | Beeinflussungsvorrichtung |
| 143 | Reinigungsgut |
| 144 | Transportvorrichtung |
| 145 | Transportrichtung |
| 146 | Einlauf |
| 148 | Auslauf |
| 149 | Antrieb |
| 150 | Reinigungszonen |
| 152 | Waschtank |
| 154 | Thermodesinfektionszone |
| 156 | Klarspülzone |
| 158 | Pumpenklarspülung |
| 160 | Frischwasserklarspülung |
| 162 | Neutralzonen |
| 164 | Trocknungszone |
| 166 | Zeitpunkt |
| 168 | Zeitpunkt |
| 170 | Hygienewertanteile pro Sekunde nach A0-Verfahren |
| 172 | Hygienewertanteile pro Sekunde nach HUE-Verfahren |
| 174 | Hygienewertanteile pro Sekunde nach SUE-Verfahren |
| 176 | Kurve |
| 178 | Kurve |
| 180 | Kurve |
| 182 | Kurve |

**Patentansprüche**

1. Verfahren zum Reinigen von Reinigungsgut (143), wobei eine Reinigungsvorrichtung (126) mit mindestens einer Reinigungskammer (128) und mindestens einer Beaufschlagungsvorrichtung (130) zum Beaufschlagen des Reinigungsguts (143) in der Reinigungskammer (128) mit mindestens einem Reinigungsfluid verwendet wird, wobei das Verfahren folgende Schritte umfasst:

   a. Vorgabe eines bei der Reinigung zu erreichenden Soll-Hygienewerts (110);
   b. zeitaufgelöste Erfassung mindestens zweier Einflussgrößen (112), welche einen Einfluss auf eine Hygienisierung des Reinigungsguts (143) haben, wobei die Einflussgrößen ausgewählt sind aus der Gruppe bestehend aus: einer Dauer der Reinigung; mindestens einer Temperatur des mindestens einen Reinigungsfluids; mindestens einer Konzentration mindestens eines Desinfektionsmittels in dem mindestens einen Reinigungsfluid; mindestens einer Konzentration mindestens eines Reinigerkonzentrats in dem mindestens einen Reinigungsfluid; mindestens einer eine Wirkung einer Klarspülung charakterisierenden Messgröße;
   c. Bestimmen von Hygienewertanteilen aus den Einflussgrößen (114) unter Verwendung eines vorgegebenen Zusammenhangs zwischen jeder Einflussgröße und deren jeweiligem Hygienewertanteil;
   d. Ermittlung eines voraussichtlichen Ist-Hygienewerts am Ende der Reinigung aus den Hygienewertanteilen (120);
   e. Vergleich des voraussichtlichen Ist-Hygienewerts mit dem Soll-Hygienewert (122); und
   f. Beeinflussung mindestens einer Einflussgröße (124) in Abhängigkeit von dem Ergebnis des Vergleichs.

2. Verfahren nach dem vorhergehenden Anspruch, wobei Verfahrensschritt e. weiterhin eine Erkennung umfasst, ob der voraussichtliche Ist-Hygienewert kleiner ist als der Soll-Hygienewert.

3. Verfahren nach einem der vorhergehenden Ansprüche, wobei der Soll-Hygienewert ausgewählt ist aus der Gruppe bestehend aus: einem bei der Reinigung insgesamt zu erreichenden AO-Wert nach DIN EN ISO 15883; einem zu erreichenden Hygienelevel nach DIN 10510, bei den Sollvorgaben von Tank und Boiler Temperaturen nach DIN 10510; einer bei der Reinigung insgesamt zu erreichenden Reduktion einer Population eines Zielkeims, insbesondere einer bei der Reinigung insgesamt zu erreichenden Reduktion der Population von Staphylococcus aureus.

**4.** Verfahren nach einem der vorhergehenden Ansprüche, wobei die mindestens zwei Einflussgrößen als erste Einflussgröße mindestens eine Temperatur des mindestens einen Reinigungsfluids umfassen sowie weiterhin mindestens eine weitere Einflussgröße.

**5.** Verfahren nach einem der vorhergehenden Ansprüche, wobei der Zusammenhang zwischen den Einflussgrößen und deren jeweiligen Hygienewertanteilen vorgegeben ist durch mindestens eine der folgenden Vorgaben: mindestens einer Kalibrationskurve, insbesondere mindestens einer Kurve zu einer Hygienewirkung, wobei die Kalibrationskurve jeweils einen Zusammenhang zwischen dem jeweiligen Hygienewertanteil der Einflussgröße und einer weiteren Einflussgröße aufzeigt, wobei die mindestens eine weitere Einflussgröße konstant gehalten wird; mindestens einer Kurvenschar mit einer Mehrzahl von Kalibrationskurven, wobei die Kalibrationskurven jeweils einen Zusammenhang zwischen dem jeweiligen Hygienewertanteil der Einflussgröße und einer weiteren Einflussgröße aufzeigen, wobei innerhalb jeder Kalibrationskurve der Kurvenschar die mindestens eine weitere Einflussgröße konstant gehalten wird, wobei sich die mindestens eine weitere Einflussgröße zwischen den Kalibrationskurven der Kurvenschar unterscheidet; mindestens einer Kalibrationstabelle, wobei die Kalibrationstabelle jeweils einen Zusammenhang zwischen dem jeweiligen Hygienewertanteil der Einflussgröße und einer weiteren Einflussgröße aufzeigt, wobei die mindestens eine weitere Einflussgröße konstant gehalten wird; mindestens einer Tabellenschar an Kalibrationstabellen mit einer Mehrzahl von Kalibrationstabellen, wobei die Kalibrationstabellen jeweils einen Zusammenhang zwischen dem jeweiligen Hygienewertanteil der Einflussgröße und einer weiteren Einflussgröße aufzeigen, wobei innerhalb jeder Kalibrationstabelle der Tabellenschar die mindestens eine weitere Einflussgröße konstant gehalten wird, wobei sich die mindestens eine weitere Einflussgröße zwischen den Kalibrationstabellen der Tabellenschar unterscheidet.

**6.** Verfahren nach einem der vorhergehenden Ansprüche, wobei die Dauer der Reinigung verändert wird, wenn bei dem Vergleich erkannt wird, dass der voraussichtliche Ist-Hygienewert von dem Soll-Hygienewert abweicht, und/oder wobei mindestens eine Konzentration mindestens eines Zusatzstoffs in dem mindestens einen Reinigungsfluid verändert wird, wenn erkannt wird, dass der voraussichtliche Ist-Hygienewert von dem Soll-Hygienewert abweicht, wobei der mindestens eine Zusatzstoff ein Reinigerkonzentrat und/oder ein Desinfektionsmittel ist.

**7.** Verfahren nach einem der vorhergehenden Ansprüche, wobei das Reinigungsgut (143) eine Mehrzahl an zu reinigenden Gütern umfasst, wobei jedem Gut ein Hygienisierungskonto zugewiesen wird, auf welchem bisherige Hygienisierungseinheiten, denen das Gut unterworfen wurde, kumuliert gespeichert werden.

**8.** Verfahren nach einem der vorhergehenden Ansprüche, wobei das Reinigungsgut (143) eine Mehrzahl an zu reinigenden Gütern umfasst, wobei das Reinigungsgut (143) mittels mindestens einer Transportvorrichtung (144) durch die mindestens eine Reinigungskammer transportiert wird, wobei die Transportvorrichtung (144) in Abschnitte unterteilt ist, wobei jedem Abschnitt ein Hygienisierungskonto zugewiesen wird, auf welchem bisherige Hygienisierungseinheiten, denen in dem jeweiligen Abschnitt aufgenommenes Gut unterworfen wurde, kumuliert gespeichert werden.

**9.** Verfahren nach dem vorhergehenden Anspruch, wobei das Verfahren mindestens einen Überprüfungsschritt umfasst, wobei der Überprüfungsschritt durchgeführt wird, solange sich das Reinigungsgut (143) innerhalb der letzten Reinigungszone in einer Transportrichtung (145) der Transportvorrichtung (144) befindet, wobei bei dem Überprüfungsschritt der voraussichtliche Ist-Hygienewert nochmals mit dem Soll-Hygienewert verglichen wird und wobei bei einem Unterschreiten des Soll-Hygienewerts mindestens eine Sicherungsmaßnahme ergriffen wird.

**10.** Reinigungsvorrichtung (126) zum Reinigen von Reinigungsgut (143), umfassend mindestens eine Reinigungskammer (128) und mindestens eine Beaufschlagungsvorrichtung (130) zum Beaufschlagen des Reinigungsguts (143) in der Reinigungskammer (128) mit mindestens einem Reinigungsfluid, wobei die Reinigungsvorrichtung (126) weiterhin umfasst:

I. mindestens einen Datenspeicher (132) zur Speicherung einer Vorgabe eines bei der Reinigung zu erreichenden Soll-Hygienewerts;
II. mindestens zwei Messvorrichtungen (134) zur zeitaufgelösten Erfassung mindestens zweier Einflussgrößen, welche einen Einfluss auf eine Hygienisierung des Reinigungsguts (143) haben, wobei die Einflussgrößen ausgewählt sind aus der Gruppe bestehend aus: einer Dauer der Reinigung; mindestens einer Temperatur des mindestens einen Reinigungsfluids; mindestens einer Konzentration mindestens eines Desinfektionsmittels in dem mindestens einen Reinigungsfluid; mindestens einer Konzentration mindestens eines Reinigerkonzentrats in dem mindestens einen Reinigungsfluid; mindestens einer eine Wirkung einer Klarspülung charakterisierenden Messgröße;

III. mindestens eine Bestimmungsvorrichtung (136) zur Bestimmung von Hygienewertanteilen aus den Einflussgrößen unter Verwendung eines vorgegebenen Zusammenhangs zwischen jeder Einflussgröße und deren jeweiligem Hygienewertanteil;

IV. mindestens eine Auswertevorrichtung (138) zur Ermittlung eines voraussichtlichen Ist-Hygienewerts an einem Ende der Reinigung aus den Hygienewertanteilen;

V. mindestens eine Vergleichsvorrichtung (140) zum Vergleich des voraussichtlichen Ist-Hygienewerts mit dem Soll-Hygienewert; und

VI. mindestens eine Beeinflussungsvorrichtung (142) zur Beeinflussung mindestens einer Einflussgröße in Abhängigkeit von dem Ergebnis des Vergleichs.

11. Reinigungsvorrichtung (126) nach dem vorhergehenden Anspruch, wobei die Reinigungsvorrichtung (126) eingerichtet ist, um ein Verfahren nach einem der vorhergehenden, ein Verfahren betreffenden, Ansprüche durchzuführen.

12. Reinigungsvorrichtung (126) nach einem der vorhergehenden, eine Reinigungsvorrichtung betreffenden, Ansprüche, wobei die Reinigungsvorrichtung (126) eine Steuerung (118) aufweist, wobei der Datenspeicher (132), die Bestimmungsvorrichtung (136), die Auswertevorrichtung (138), die Vergleichsvorrichtung (140) und die Beeinflussungsvorrichtung (142) Bestandteile der Steuerung (118) sind.

13. Reinigungsvorrichtung (126) nach dem vorhergehenden Anspruch, wobei die Steuerung (118) mindestens eine Datenverarbeitungsvorrichtung, insbesondere mindestens einen Prozessor, umfasst, wobei die Datenverarbeitungsvorrichtung programmtechnisch eingerichtet ist, um den Einflussgrößen aufgrund des vorgegebenen Zusammenhangs jeweils einen Hygienewertanteil zuzuweisen und um den voraussichtlichen Ist-Hygienewert am Ende der Reinigung aus den Hygienewertanteilen zu bestimmen.

14. Reinigungsvorrichtung (126) nach dem vorhergehenden Anspruch, wobei die Datenverarbeitungsvorrichtung weiterhin programmtechnisch eingerichtet ist, um den voraussichtlichen Ist-Hygienewert mit dem Soll-Hygienewert zu vergleichen und um zu erkennen, ob der voraussichtliche Ist-Hygienewert kleiner ist als der Soll-Hygienewert.

15. Reinigungsvorrichtung (126) nach dem vorhergehenden Anspruch, wobei die Datenverarbeitungsvorrichtung weiterhin programmtechnisch eingerichtet ist, um die mindestens eine Einflussgröße in Abhängigkeit von dem Ergebnis des Vergleichs zu beeinflussen.

16. Reinigungsvorrichtung (126) nach einem der vorhergehenden, eine Reinigungsvorrichtung betreffenden, Ansprüche, wobei die Messvorrichtungen (134) mindestens zwei Sensoren umfassen, ausgewählt aus der Gruppe bestehend aus: mindestens einem Temperatursensor zur Erfassung mindestens einer Temperatur des mindestens einen Reinigungsfluids; mindestens einem Konzentrationssensor zur Erfassung mindestens einer Konzentration mindestens eines Desinfektionsmittels in dem mindestens einen Reinigungsfluid; mindestens einem Konzentrationssensor zur Erfassung mindestens einer Konzentration mindestens eines Reinigerkonzentrats in dem mindestens einen Reinigungsfluid; mindestens einem pH-Sensor zur Erfassung mindestens eines pH Werts des mindestens einen Reinigungsfluids.

17. Reinigungsvorrichtung (126) nach einem der vorhergehenden, eine Reinigungsvorrichtung betreffenden, Ansprüche, wobei die Reinigungsvorrichtung (126) ausgewählt ist aus der Gruppe bestehend aus einer Transportspülmaschine und einer Programmspülmaschine.

**Claims**

1. Method for cleaning items (143) to be cleaned, wherein a cleaning device (126) having at least one cleaning chamber (128) and at least one application device (130) for applying at least one cleaning fluid to the items (143) to be cleaned in the cleaning chamber (128) is used, wherein the method comprises the following steps:

a. prespecifying a target hygiene value (110) which is to be achieved with the cleaning;

b. time-resolved recording of at least two influencing variables (112) which have an influence on a hygienization of the items (143) to be cleaned, wherein the influencing variables are selected from the group consisting of: a duration of the cleaning; at least one temperature of the at least one cleaning fluid; at least one concentration of at least one disinfectant in the at least one cleaning fluid; at least one concentration of at least one detergent concentrate in the at least one cleaning fluid; at least one measurement variable which is characteristic of an effect of a final rinse operation;

c. determining hygiene value shares from the influencing variables (114) using a prespecified relationship between each influencing variable and the respective hygiene value share of said influencing variable;

    d. ascertaining an expected actual hygiene value at the end of the cleaning from the hygiene value shares (120) ;

    e. comparing the expected actual hygiene value with the target hygiene value (122); and

    f. influencing at least one influencing variable (124) depending on the result of the comparison.

2. Method according to the preceding claim, wherein method step e. further comprises identifying whether the expected actual hygiene value is lower than the target hygiene value.

3. Method according to either of the preceding claims, wherein the target hygiene value is selected from the group consisting of: an AO value according to DIN EN ISO 15883 which is to be achieved overall with the cleaning; a hygiene level which is to be achieved according to DIN 10510, at the desired prespecifications for tank and boiler temperatures according to DIN 10510; a reduction in a population of a target germ which is to be achieved overall with the cleaning, in particular a reduction in the population of Staphylococcus aureus which is to be achieved overall with the cleaning.

4. Method according to one of the preceding claims, wherein the at least two influencing variables comprise, as first influencing variable, at least one temperature of the at least one cleaning fluid and furthermore at least one further influencing variable.

5. Method according to one of the preceding claims, wherein the relationship between the influencing variables and the respective hygiene value shares of said influencing variables is prespecified by at least one of the following prespecifications: at least one calibration curve, in particular at least one curve relating to a hygiene effect, wherein the calibration curve respectively indicates a relationship between the respective hygiene value share of the influencing variable and a further influencing variable, wherein the at least one further influencing variable is kept constant; at least one family of curves comprising a plurality of calibration curves, wherein the calibration curves each indicate a relationship between the respective hygiene value share of the influencing variable and a further influencing variable, wherein the at least one further influencing variable is kept constant within each calibration curve of the family of curves, wherein the at least one further influencing variable distinguishes between the calibration curves of the family of curves; at least one calibration table, wherein the calibration table respectively indicates a relationship between the respective hygiene value share of the influencing variable and a further influencing variable, wherein the at least one further influencing variable is kept constant; at least one family of calibration tables comprising a plurality of calibration tables, wherein the calibration tables each indicate a relationship between the respective hygiene value share of the influencing variable and a further influencing variable, wherein the at least one further influencing variable is kept constant within each calibration table of the family of tables, wherein the at least one further influencing variable distinguishes between the calibration tables of the family of tables.

6. Method according to one of the preceding claims, wherein the duration of the cleaning is changed when it is identified during the comparison that the expected actual hygiene value differs from the target hygiene value, and/or wherein at least one concentration of at least one additive in the at least one cleaning fluid is changed when it is identified that the expected actual hygiene value differs from the target hygiene value, wherein the at least one additive is a detergent concentrate and/or a disinfectant.

7. Method according to one of the preceding claims, wherein the items (143) to be cleaned comprise a plurality of items to be cleaned, wherein each item is allocated a hygienization account in which previous hygienization units to which the item has been subjected are cumulatively stored.

8. Method according to one of the preceding claims, wherein the items (143) to be cleaned comprise a plurality of items to be cleaned, wherein the items (143) to be cleaned are transported through the at least one cleaning chamber by means of at least one transportation device (144), wherein the transportation device (144) is subdivided into sections, wherein each section is allocated a hygienization account in which previous hygienization units to which items held in the respective section have been subjected are cumulatively stored.

9. Method according to the preceding claim, wherein the method comprises at least one checking step, wherein the checking step is carried out while the items (143) to be cleaned are located within the last cleaning zone in a transportation direction (145) of the transportation device (144), wherein, during the checking step, the expected actual hygiene value is once again compared with the target hygiene value, and wherein at least one safety measure is taken when the target hygiene value is undershot.

10. Cleaning device (126) for cleaning items (143) to be cleaned, comprising at least one cleaning chamber (128) and at least one application device (130) for applying at least one cleaning fluid to the items (143) to be cleaned in the cleaning chamber (128), wherein the cleaning device (126) further comprises:

I. at least one data memory (132) for storing a prespecification for a target hygiene value which is to be achieved with the cleaning;
II. at least two measuring devices (134) for timeresolved recording of at least two influencing variables which have an influence on a hygienization of the items (143) to be cleaned, wherein the influencing variables are selected from the group consisting of: a duration of the cleaning; at least one temperature of the at least one cleaning fluid; at least one concentration of at least one disinfectant in the at least one cleaning fluid; at least one concentration of at least one detergent concentrate in the at least one cleaning fluid; at least one measurement variable which is characteristic of an effect of a final rinse operation;
III. at least one determining device (136) for determining hygiene value shares from the influencing variables using a prespecified relationship between each influencing variable and the respective hygiene value share of said influencing variable;
IV. at least one evaluating device (138) for ascertaining an expected actual hygiene value at the end of the cleaning from the hygiene value shares;
V. at least one comparing device (140) for comparing the expected actual hygiene value with the target hygiene value; and
VI. at least one influencing device (142) for influencing at least one influencing variable depending on the result of the comparison.

**11.** Cleaning device (126) according to the preceding claim, wherein the cleaning device (126) is designed to carry out a method according to one of the preceding claims which relates to a method.

**12.** Cleaning device (126) according to one of the preceding claims which relates to a cleaning device, wherein the cleaning device (126) has a controller (118), wherein the data memory (132), the determining device (136), the evaluating device (138), the comparing device (140) and the influencing device (142) are constituent parts of the controller (118).

**13.** Cleaning device (126) according to the preceding claim, wherein the controller (118) comprises at least one data processing device, in particular at least one processor, wherein the data processing device is designed in terms of programming to allocate in each case one hygiene value share to the influencing variables on the basis of the prespecified relationship and to determine the expected actual hygiene value at the end of the cleaning from the hygiene value shares.

**14.** Cleaning device (126) according to the preceding claim, wherein the data processing device is further designed in terms of programming to compare the expected actual hygiene value with the target hygiene value and to identify whether the expected actual hygiene value is lower than the target hygiene value.

**15.** Cleaning device (126) according to the preceding claim, wherein the data processing device is further designed in terms of programming to influence the at least one influencing variable depending on the result of the comparison.

**16.** Cleaning device (126) according to one of the preceding claims which relates to a cleaning device, wherein the measuring devices (134) comprise at least two sensors selected from the group consisting of: at least one temperature sensor for recording at least one temperature of the at least one cleaning fluid; at least one concentration sensor for recording at least one concentration of at least one disinfectant in the at least one cleaning fluid; at least one concentration sensor for recording at least one concentration of at least one detergent concentrate in the at least one cleaning fluid; at least one pH sensor for recording at least one pH value of the at least one cleaning fluid.

**17.** Cleaning device (126) according to one of the preceding claims which relates to a cleaning device, wherein the cleaning device (126) is selected from the group consisting of a conveyor washer and a box-type washer.

**Revendications**

**1.** Procédé de nettoyage d'articles à nettoyer (143), un dispositif de nettoyage (126) étant utilisé qui comprend au moins une chambre de nettoyage (128) et au moins un dispositif d'application (130) destiné à appliquer au moins un fluide de nettoyage sur les articles à nettoyer (143) dans la chambre de nettoyage (128), le procédé comprenant les étapes suivantes :

a. spécifier une valeur d'hygiène cible (110) à atteindre lors du nettoyage ;
b. effectuer une détection résolue dans le temps d'au moins deux grandeurs influentes (112) qui influe sur l'hygiénisation des articles à nettoyer (143) ; les grandeurs influentes étant choisies dans le groupe comprenant : une durée du nettoyage ; au moins une température de l'au moins un fluide de nettoyage ; au moins une concentration d'au moins un désinfectant dans l'au moins un fluide de nettoyage ; au moins une concentration d'au moins un concentré de nettoyage dans l'au moins un fluide de nettoyage ; au moins une grandeur de mesure caractérisant un effet d'un rinçage final ;

c. déterminer des proportions de valeur d'hygiène à partir des grandeurs influentes (114) à l'aide d'une relation spécifiée entre chaque grandeur influente et sa proportion de valeur d'hygiène respective ;

d. déterminer une valeur d'hygiène réelle probable à la fin du nettoyage à partir des proportions de valeur d'hygiène (120) ;

e. comparer la valeur d'hygiène réelle probable à la valeur d'hygiène cible (122) ; et

f. influer sur au moins une grandeur influente (124) en fonction du résultat de la comparaison.

2. Procédé selon la revendication précédente, l'étape de procédé e. comprenant en outre une détection pour savoir si la valeur d'hygiène réelle probable est inférieure à la valeur d'hygiène cible.

3. Procédé selon l'une des revendications précédentes, la valeur d'hygiène cible étant choisie dans le groupe comprenant : une valeur A0 à atteindre globalement pendant le nettoyage selon DIN EN ISO 15883 ; un niveau d'hygiène à atteindre selon DIN 10510, avec les spécifications cibles pour les températures de réservoir et de chaudière selon DIN 10510 ; une réduction de la population d'un germe cible à atteindre globalement lors du nettoyage, en particulier une réduction de la population de Staphylococcus aureus à atteindre globalement lors du nettoyage.

4. Procédé selon l'une des revendications précédentes, les au moins deux grandeurs influentes comprenant au moins une température de l'au moins un fluide de nettoyage et également au moins une autre grandeur influente.

5. Procédé selon l'une des revendications précédentes, la relation entre les grandeurs influentes et leurs proportions de valeur d'hygiène respectives étant spécifiée par l'une au moins des spécifications suivantes : au moins une courbe d'étalonnage, en particulier au moins une courbe d'effet hygiénique, la courbe d'étalonnage montrant une relation entre la proportion de valeur d'hygiène respective de la grandeur influente et une autre grandeur influente, l'au moins une autre grandeur influente étant maintenue constante ; au moins une famille de courbes comportant une pluralité de courbes d'étalonnage, les courbes d'étalonnage montrant chacune une relation entre la proportion de valeur d'hygiène respective de la grandeur influente et une autre grandeur influente, l'au moins une autre grandeur influente étant maintenue constante à l'intérieur de chaque courbe d'étalonnage de la famille de courbes, l'au moins une autre grandeur influente se distinguant entre les courbes d'étalonnage de la famille de courbes ; au moins une table d'étalonnage, la table d'étalonnage montrant une relation entre la proportion de valeur d'hygiène respective de la grandeur influente et une autre grandeur influente, l'au moins une autre grandeur influente étant maintenue constante ; au moins une famille de tables d'étalonnage comportant une pluralité de tables d'étalonnage, les tables d'étalonnage montrant chacune une relation entre la proportion de valeur d'hygiène respective de la grandeur influente et une autre grandeur influente, l'au moins une autre grandeur influente étant maintenue constante dans chaque table d'étalonnage de la famille de tables, l'au moins une autre grandeur influente se distinguant entre les tables d'étalonnage de la famille de tables.

6. Procédé selon l'une des revendications précédentes, la durée du nettoyage étant modifiée s'il est détecté lors de la comparaison que la valeur d'hygiène réelle probable s'écarte de la valeur d'hygiène cible, et/ou au moins une concentration d'au moins un additif dans l'au moins un fluide de nettoyage étant modifiée s'il est détecté que la valeur d'hygiène réelle probable s'écarte de la valeur d'hygiène cible, l'au moins un additif étant un concentré de nettoyage et/ou un désinfectant.

7. Procédé selon l'une des revendications précédentes, les articles à nettoyer (143) comprenant une pluralité d'articles à nettoyer, chaque article étant associé à un compte d'hygiénisation sur lequel sont mémorisées de manière cumulée des unités d'hygiénisation précédentes auxquelles l'article a été soumis.

8. Procédé selon l'une des revendications précédentes, les articles à nettoyer (143) comprenant une pluralité d'articles à nettoyer, les articles à nettoyer (143) étant transportés à travers l'au moins une chambre de nettoyage au moyen d'au moins un dispositif de transport (144), le dispositif de transport (144) étant divisé en portions, chaque portion étant associée à un compte d'hygiénisation sur lequel des unités d'hygiénisation précédentes, auxquelles les articles reçus dans la portion respective ont été soumis, sont mémorisées de manière cumulée.

9. Procédé selon la revendication précédente, le procédé comprenant au moins une étape de vérification, l'étape de vérification étant exécutée tant que l'article à nettoyer (143) se trouve dans la dernière zone de nettoyage dans une direction de transport (145) du dispositif de transport (144), la valeur d'hygiène réelle probable étant à nouveau comparée à la valeur d'hygiène cible lors de l'étape de vérification, et au moins une mesure de sécurité étant prise si la valeur d'hygiène cible n'est pas atteinte.

10. Dispositif de nettoyage (126) destiné à nettoyer des articles à nettoyer (143), le dispositif de nettoyage comprenant au moins une chambre de nettoyage (128) et au moins un dispositif d'application (130) destiné à appliquer au moins un fluide de nettoyage sur les articles à nettoyer (143) dans la chambre de nettoyage (128), le dispositif de nettoyage (126) comprenant en outre :

I. au moins une mémoire de données (132) destinée à mémoriser une spécification d'une valeur d'hygiène cible à atteindre pendant le nettoyage ;

II. au moins deux dispositifs de mesure (134) destinés à effectuer la détection résolue en temps d'au moins deux grandeurs influentes qui influent sur l'hygiénisation des articles à nettoyer (143) ; les grandeurs influentes étant choisies dans le groupe comprenant : une durée du nettoyage ; au moins une température de l'au moins un fluide de nettoyage ; au moins une concentration d'au moins un désinfectant dans l'au moins un fluide de nettoyage ; au moins une concentration d'au moins un concentré de nettoyage dans l'au moins un fluide de nettoyage ; au moins une grandeur de mesure caractérisant un effet d'un rinçage final ;

III. au moins un dispositif de détermination (136) destiné à déterminer les proportions de valeur d'hygiène à partir des grandeurs influentes à l'aide d'une relation spécifiée entre chaque grandeur influente et sa proportion de valeur d'hygiène respective ;

IV. au moins un dispositif d'évaluation (138) destiné à déterminer une valeur d'hygiène réelle probable à la fin du nettoyage à partir des proportions de valeur d'hygiène ;

V. au moins un dispositif de comparaison (140) destiné à comparer la valeur d'hygiène réelle probable à la valeur d'hygiène cible ; et

VI. au moins un dispositif d'influence (142) destiné à influer sur au moins une grandeur influente en fonction du résultat de la comparaison.

11. Dispositif de nettoyage (126) selon la revendication précédente, le dispositif de nettoyage (126) étant conçu pour mettre en œuvre un procédé selon l'une des revendications précédentes relatives à un procédé.

12. Dispositif de nettoyage (126) selon l'une des revendications précédentes relatives à un dispositif de nettoyage, le dispositif de nettoyage (126) comportant une commande (118), la mémoire de données (132), le dispositif de détermination (136), le dispositif d'évaluation (138), le dispositif de comparaison (140) et le dispositif d'influence (142) étant des composants de la commande (118).

13. Dispositif de nettoyage (126) selon la revendication précédente, la commande (118) comprenant au moins un dispositif de traitement de données, en particulier au moins un processeur, le dispositif de traitement de données étant adapté en termes de technologie de programme pour attribuer une proportion de valeur d'hygiène à chacune des grandeurs influentes sur la base de la relation spécifiée et pour déterminer la valeur d'hygiène réelle probable à la fin du nettoyage à partir des proportions de valeur d'hygiène.

14. Dispositif de nettoyage (126) selon la revendication précédente, le dispositif de traitement de données étant également adapté en termes de technologie de programme pour comparer la valeur d'hygiène réelle probable à la valeur d'hygiène cible et pour détecter si la valeur d'hygiène réelle probable est inférieure à la valeur d'hygiène cible.

15. Dispositif de nettoyage (126) selon la revendication précédente, le dispositif de traitement de données étant également adapté en termes de technologie de programme pour influer sur l'au moins une grandeur influente en fonction du résultat de la comparaison.

16. Dispositif de nettoyage (126) selon l'une des revendications précédentes relatives à un dispositif de nettoyage, les dispositifs de mesure (134) comprenant au moins deux capteurs choisis dans le groupe comprenant : au moins un capteur de température destiné à détecter au moins une température de l'au moins un fluide de nettoyage ; au moins un capteur de concentration destiné à détecter au moins une concentration d'au moins un désinfectant dans l'au moins un fluide de nettoyage ; au moins un capteur de concentration destiné à détecter au moins une concentration d'au moins un concentré de nettoyage dans l'au moins un fluide de nettoyage ; au moins un capteur de pH destiné à détecter au moins une valeur de pH de l'au moins un fluide de nettoyage.

17. Dispositif de nettoyage (126) selon l'une des revendications précédentes relatives à un dispositif de nettoyage, le dispositif de nettoyage (126) étant choisi dans le groupe comprenant un lave-vaisselle à convoyeur et un lave-vaisselle à programme.

```
┌─────────────┐
│     110     │
└─────────────┘
       │
       ▽
┌─────────────┐
│     112     │
└─────────────┘
       │
       ▽
┌─────────────┐
│     114     │
└─────────────┘
       │
       ▽
┌─────────────┐
│     116     │
└─────────────┘
       │
       ▽
┌─────────────┐
│     120     │
└─────────────┘
       │
       ▽
┌─────────────┐
│     122     │
└─────────────┘
       │
       ▽
┌─────────────┐
│     124     │
└─────────────┘
```

FIG. 1

FIG. 2

FIG. 3

FIG. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102004056052 A1
- DE 102007025263 A1
- DE 102013220035 A1
- EP 2053959 B1
- DE 102006014464 B3
- DE 102006019546 A1
- EP 1886615 B1
- DE 102013203933 A1
- DE 102014102970 A1
- DE 102009024569 A1
- DE 102011007507 A1
- DE 102012213271 A1
- DE 102008024543 A1
- WO 2011062790 A2
- WO 2006097294 A1

- EP 2241240 A1
- DE 102013226637 A1
- US 6615850 B1
- DE 102004049392 A1
- DE 102007021245 A1
- US 4439242 A
- US 5603233 A
- US 5820691 A
- US 3896827 A
- EP 2228000 A2
- US 20160309981 A1
- EP 1824373 B1
- EP 0621810 B1
- US 20160206767 A1
- DE 69519474 T2